(19) Europäisches Patentamt
European Patent Office
Office européen des brevets

(11) EP 1 595 869 A1

(12) **EUROPEAN PATENT APPLICATION**
published in accordance with Art. 158(3) EPC

(43) Date of publication:
**16.11.2005 Bulletin 2005/46**

(21) Application number: **04713192.5**

(22) Date of filing: **20.02.2004**

(51) Int Cl.[7]: **C07D 239/42**, C07D 401/04, C07D 401/14, C07D 405/14, C07D 409/14, C07D 417/14, A61K 31/506, A61P 29/00, A61P 37/06, A61P 37/08, A61P 43/00

(86) International application number:
**PCT/JP2004/001962**

(87) International publication number:
**WO 2004/074260 (02.09.2004 Gazette 2004/36)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **21.02.2003 JP 2003044174**
**05.09.2003 JP 2003314058**

(71) Applicant: **KYOWA HAKKO KOGYO CO., LTD.**
**Chiyoda-ku, Tokyo 100-8185 (JP)**

(72) Inventors:
- **OHSHIMA, Etsuo (JP)**
- **MIYAMA, Motoki (JP)**
- **YANAGAWA, Koji (JP)**
- **ARATAKE, Seiji (JP)**
- **MIKI, Ichiro (JP)**
- **KOBAYASHI, Katsuya (JP)**
- **SATO, Takashi (JP)**
- **KAWABE, Ari (JP)**
- **TAGAYA, Miho (JP)**

(74) Representative: **Vossius & Partner**
**Siebertstrasse 4**
**81675 München (DE)**

(54) **PYRIMIDINE DERIVATIVES**

(57) The present invention provides a pyrimidine derivative represented by Formula (I)

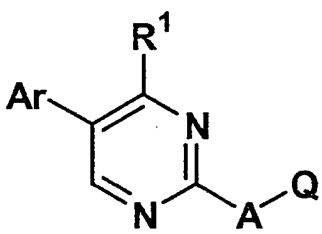

(I)

[wherein Ar represents substituted or unsubstituted aryl, etc., $R^1$ represents $-NR^2R^3$ (wherein $R^2$ represents a hydrogen atom or substituted or unsubstituted lower alkyl and $R^3$ represents substituted or unsubstituted lower alkyl, substituted or unsubstituted aralkyl, etc.), etc. A represents a single bond, Formula (III)

EP 1 595 869 A1

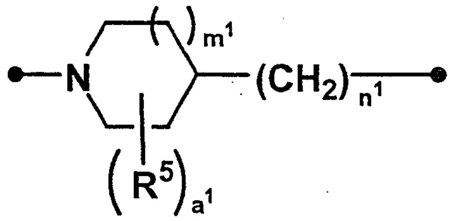

(III)

(wherein $m^1$ represents an integer of 0 to 2, $n^1$ represents an integer of 0 to 4, $a^1$ represents a number ranging from 0 to a substitutable number, $R^5$ represents substituted or unsubstituted lower alkyl, etc. and each $R^5$ may be the same or different when $a^1$ is 2 or more), etc. and Q represents $-NR^6R^7$ (wherein $R^6$ and $R^7$ may be the same or different and each represents a hydrogen atom, a substituted or unsubstituted heteroalicyclic group, etc.), a substituted or unsubstituted heteroalicyclic group, substituted or unsubstituted piperidin-4-ylamino, etc.], or quaternary ammonium salts thereof, or pharmaceutically acceptable salts thereof which have anti-inflammatory activities, modulating activities on TARC and/or MDC functions and the like, and are useful for treating and/or preventing diseases which is related to T cells such as allergic diseases, autoimmune diseases, transplant rejection, etc.

## Description

### Technical Field

The present invention relates to pyrimidine derivatives, or quaternary ammonium salts thereof, or pharmaceutically acceptable salts thereof which have anti-inflammatory activities such as cellular infiltration inhibitory activities, etc., regulatory activities on the functions of thymus and activation-regulated chemokine [TARC; CC chemokine ligand 17 (CCL17)] and/or macrophage-derived chemokine [MDC; CC chemokine ligand 22 (CCL22)] and the like, and are useful for, for example, treating and/or preventing various diseases which are related to T cells, such as allergic diseases, autoimmune diseases, etc.

### Background Art

Compounds containing a pyrimidine skeleton in the structure thereof are disclosed as an anti-inflammatory agent (WO2000/43369, WO97/09325, WO98/38171), a therapeutic agent for cardiac dysfunction (WO2001/27107), a therapeutic agent for neurodegenerative or neuropathic disease (WO99/19305, EP0459819), a therapeutic agent for central nervous system disease (EP0372934), a cytokine inhibitor [Japanese Translation of PCT International Application No. 503017/1995, Japanese Published Unexamined Patent Application (Kokai) No. 97189/2002], an inhibitor of prostaglandin endoperoxide synthase-2 (PGHS-2), cyclooxygenase-2 (COX-2), cytokine specific binding protein (CSBP), RK or p38 kinase (Japanese Translation of PCT International Application No. 512555/1998), an anti-inflammatory agent or a therapeutic agent for cytokine p38/mitogen-activated protein kinase (MAP kinase)-mediated disease (Japanese Translation of PCT International Application No. 500122/2001), a therapeutic agent for cytokine-mediated disease (US6268370, Japanese Translation of PCT International Application No. 506532/2000), an anti-allergy agent (WO2002/20012), a therapeutic agent for autoimmune disease (W02002/14314), an immunosuppressive agent (Japanese Translation of PCT International Application No. 525406/2001), a protein kinase inhibitor (WO2002/22602), a p38 kinase inhibitor (Japanese Translation of PCT International Application No. 508754/2002), a factor Xa inhibitor (US5916891, WO2001/32628), a germicide [Japanese Published Unexamined Patent Application (Kokai) No. 33611/1995], a glycogen synthase inhibitor (WO99/65897, WO2002/20495), a leukotriene antagonist (Japanese Translation of PCT International Application No. 508845/1993), a therapeutic agent for very late activation antigen (VLA)-mediated disease (WO2002/08201, WO2002/08203), a farnesyl protein transferase inhibitor (US5872136), a neuronal NO synthase inhibitor (Japanese Translation of PCT International Application No. 504798/1996), an antitumor agent (WO2002/20500), an aurora-2 kinase inhibitor (WO2001/21597), a tyrosine kinase inhibitor (WO2001/17995), SRC kinase inhibitor (WO2001/00207, W02001/00214), an antiviral agent (WO99/41253), and a CC chemokine receptor-4 (CCR4) function modulator (W02002/30358), respectively.

TARC has been found as a T-cell chemotactic factor *[Journal* of *Biological Chemistry,* vol. 271, p. 21514 (1996)] and MDC has been found as a monocyte chemotactic factor [*Journal of Experimental Medicine*, vol. 185, p. 1595 (1997)]. In particular, TARC is assumed to be involved in allergic diseases, since it is produced from monocytes stimulated by T Helper 2 (Th2) cytokine *[Journal of Biological Chemistry,* vol. 271, p. 21514 (1996)]. Further analyses have shown that TARC and MDC are CCR4 ligands *[Journal* of *Biological Chemistry,* vol. 272, p. 15036 (1997); *Journal of Biological Chemistry,* vol. 273, p. 1764 (1998)].

CCR4 has been cloned as a receptor expressed in T cells and thymocytes *[Biochemical and Biophysical Research Communications,* vol. 218, p. 337 (1996)] and further investigations have reported that CCR4 is mainly expressed in "Th2-type" T cells *[Journal of Experimental Medicine,* vol. 187, p. 875 (1998); *Journal of Immunology,* vol. 161, p. 5027 (1998)].

### Disclosure of Invention

An object of the present invention is to provide pyrimidine derivatives, or quaternary ammonium salts thereof, or pharmaceutically acceptable salts thereof which have anti-inflammatory activities (for example, cellular infiltration inhibitory activities), modulating activities on TARC and/or MDC functions (for example, inhibitory activities against binding of TARC and/or MDC to T cells), or the like, and are useful for treating and/or preventing diseases which is related to T cells such as allergic diseases, autoimmune diseases, or transplant rejection, as well as preventing cancer metastasis. Examples of the diseases which is related to T cells include asthma, allergic rhinitis, chronic rhinitis, pollinosis, conjunctivitis, urticaria, psoriasis, atopic dermatitis, cutaneous pruritus, cutaneous candidiasis, oral candidiasis, articular rheumatism, various collagen diseases, systemic lupus erythematosus, Sjögren syndrome, cellular rejection after in transplantation, cancer, leukemia such as adult T cell leukemia (ATL), eosinophilic pneumonia, eosinophilic sinusitis, rhinitis with eosinophilia, interstitial cystitis, endometriosis, Churg-Strauss syndrome, septicemia, pain, neuralgia, insulin-dependent diabetes mellitus (IDDM), mycosis fungoides, contact dermatitis, and cutaneous T cell lymphoma.

The present invention relates to Items (1) to (29) below.

(1) A pyrimidine derivative represented by Formula (I)

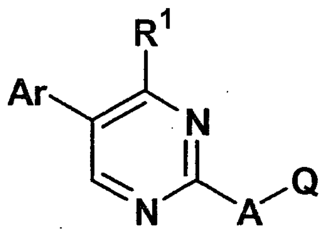

(I)

[wherein Ar represents substituted or unsubstituted aryl or a substituted or unsubstituted heteroaromatic group, $R^1$ represents $-NR^2R^3$ (wherein $R^2$ represents a hydrogen atom or substituted or unsubstituted lower alkyl, and $R^3$ represents substituted or unsubstituted lower alkyl, substituted or unsubstituted aralkyl or substituted or unsubstituted heteroaromatic alkyl) or Formula (II)

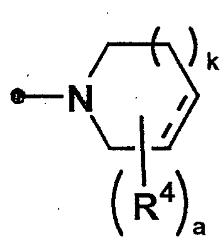

(II)

(wherein --- represents a single or double bond, k represents an integer of 0 to 4, a represents a number ranging from 0 to a substitutable number, $R^4$ represents substituted or unsubstituted lower alkyl, and each $R^4$ may be the same or different when a is 2 or more),
A represents a single bond, Formula (III)

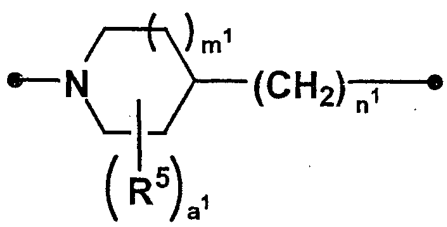

(III)

(wherein $m^1$ represents an integer of 0 to 2, $n^1$ represents an integer of 0 to 4, $a^1$ represents a number ranging from 0 to a substitutable number, $R^5$ represents substituted or unsubstituted lower alkyl, substituted or unsubstituted aralkyl, a substituted or unsubstituted heteroalicyclic group, or substituted or unsubstituted heteroaromatic alkyl, and each $R^5$ may be the same or different when $a^1$ is 2 or more) or Formula (IV)

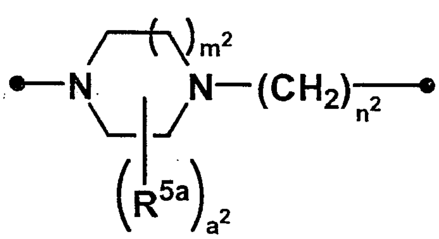

(IV)

(wherein $a^2$ represents a number ranging from 0 to a substitutable number, and $m^2$, $n^2$ and $R^{5a}$ have the same meanings as $m^1$, $n^1$, and $R^5$ defined above, respectively), and

(a) when A represents Formula (III) or (IV),
Q represents $-NR^6R^7$ (wherein $R^6$ and $R^7$ may be the same or different and each represents a hydrogen atom, substituted or unsubstituted lower alkyl, substituted or unsubstituted aralkyl, a substituted or unsubstituted heteroalicyclic group, substituted or unsubstituted heteroalicyclic alkyl or substituted or unsubstituted heteroaromatic alkyl) or a substituted or unsubstituted heteroalicyclic group,
(b) when A represents a single bond,
Q represents substituted or unsubstituted piperidin-4-ylamino, substituted or unsubstituted perhydroazepin-4-ylamino or $-N(R^8)-(CH_2)_p-NR^9R^{10}$ (wherein p represents 1, 2 or 4, $R^8$ represents a hydrogen atom or substituted or unsubstituted lower alkyl, and $R^9$ and $R^{10}$ may be the same or different and each represents a hydrogen atom, substituted or unsubstituted lower alkyl, substituted or unsubstituted aralkyl, a substituted or unsubstituted heteroalicyclic group, substituted or unsubstituted heteroalicyclic alkyl or substituted or unsubstituted heteroaromatic alkyl, or $R^9$ and $R^{10}$ are combined together with the adjacent nitrogen atom thereto to form a substituted or unsubstituted heteroalicyclic group], or a quaternary ammonium salt thereof, or a pharmaceutically acceptable salt thereof.

(2) The pyrimidine derivative, or the quaternary ammonium salt thereof, or the pharmaceutically acceptable salt thereof according to (1), wherein $R^1$ is $-NR^2R^{3A}$ (wherein $R^2$ has the same meaning as defined above, and $R^{3A}$ represents substituted or unsubstituted aralkyl or substituted or unsubstituted heteroaromatic alkyl).
(3) The pyrimidine derivative, or the quaternary ammonium salt thereof, or the pharmaceutically acceptable salt thereof according to (2), wherein $R^{3A}$ is substituted aralkyl.
(4) The pyrimidine derivative, or the quaternary ammonium salt thereof, or the pharmaceutically acceptable salt thereof according to any of (1) to (3), wherein A represents Formula (III)

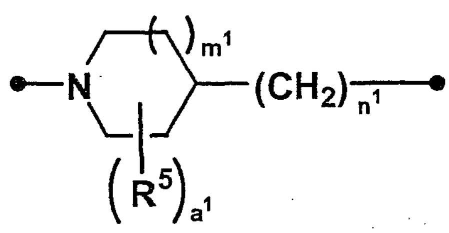

(III)

(wherein $m^1$, $n^1$, $a^1$ and $R^5$ have the same meanings as defined above, respectively) or Formula (IV)

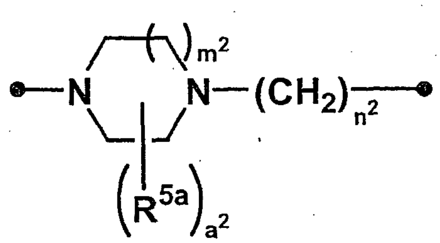

(IV)

(wherein $m^2$, $n^2$, $a^2$ and $R^{5a}$ have the same meanings as defined above, respectively).
(5) The pyrimidine derivative, or the quaternary ammonium salt thereof, or the pharmaceutically acceptable salt thereof according to (4), wherein $m^1$ is 1 or 2 and $m^2$ is 1.
(6) The pyrimidine derivative, or the quaternary ammonium salt thereof, or the pharmaceutically acceptable salt thereof according to any of (1) to (3), wherein A is Formula (IIIA)

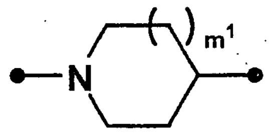

(IIIA)

(wherein $m^1$ has the same meaning as defined above).

(7) The pyrimidine derivative, or the quaternary ammonium salt thereof, or the pharmaceutically acceptable salt thereof according to (6), wherein $m^1$ is 1.

(8) The pyrimidine derivative, or the quaternary ammonium salt thereof, or the pharmaceutically acceptable salt thereof according to any of (1) to (7), wherein Q is $-NR^6R^7$ (wherein $R^6$ and $R^7$ have the same meanings as defined above, respectively), substituted or unsubstituted 1,2,5,6-tetrahydropyridin-1-yl, substituted or unsubstituted 1,2,3,4-tetrahydroisoquinolin-2-yl or Formula (V)

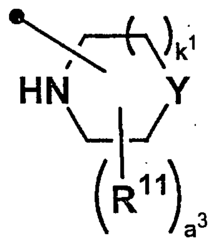

(V)

{wherein $k^1$ represents an integer of 1 to 4, $a^3$ represents a number ranging 0 to a substitutable number, Y represents a single bond, -S-, -O-, -C(=O)- or $-N(R^{12})-$ (wherein $R^{12}$ represents a hydrogen atom or substituted or unsubstituted alkanoyl), $R^{11}$ represents a hydrogen atom, substituted or unsubstituted lower alkyl, substituted or unsubstituted lower alkenyl, substituted or unsubstituted lower alkynyl, substituted or unsubstituted mono(lower alkyl)amino, substituted or unsubstituted di(lower alkyl)amino, substituted or unsubstituted lower alkoxy, hydroxy, substituted or unsubstituted lower alkanoyl, substituted or unsubstituted aryl, substituted or unsubstituted aralkyl, substituted or unsubstituted aryloxy, substituted or unsubstituted aralkyloxy, substituted or unsubstituted aroyl, substituted or unsubstituted lower alkoxycarbonyl, substituted or unsubstituted mono(lower alkyl)aminocarbonyl, substituted or unsubstituted di(lower alkyl)aminocarbonyl, cyano, carboxy, carbamoyl, amino, nitro, substituted or unsubstituted lower alkylsulfinyl, substituted or unsubstituted lower alkylsulfonyl, mercapto or lower alkylthio,
and when $a^3$ is 2 or more, each $R^{11}$ may be the same or different.

(9) The pyrimidine derivative, or the quaternary ammonium salt thereof, or the pharmaceutically acceptable salt thereof according to any of (1) to (7), wherein Q is substituted or unsubstituted piperidino.

(10) The pyrimidine derivative, or the quaternary ammonium salt thereof, or the pharmaceutically acceptable salt thereof according to any of (1) to (3), wherein A is a single bond.

(11) The pyrimidine derivative, or the quaternary ammonium salt thereof, or the pharmaceutically acceptable salt thereof according to any of (1) to (10), wherein Ar is substituted or unsubstituted aryl.

(12) The pyrimidine derivative, or the quaternary ammonium salt thereof, or the pharmaceutically acceptable salt thereof according to any of (1) to (10), wherein Ar is substituted or unsubstituted phenyl, substituted or unsubstituted naphthyl, substituted or unsubstituted biphenyl, substituted or unsubstituted thienyl, substituted or unsubstituted thiazolyl, substituted or unsubstituted benzofuranyl, substituted or unsubstituted furyl or substituted or unsubstituted pyridyl.

(13) The pyrimidine derivative, or the quaternary ammonium salt thereof, or the pharmaceutically acceptable salt thereof according to any of (1) to (12), wherein $R^2$ is a hydrogen atom.

(14) The pyrimidine derivative, or the quaternary ammonium salt thereof, or the pharmaceutically acceptable salt thereof according to any of (1) to (13), wherein the quaternary ammonium salt is formed by addition of Z-El (wherein Z represents substituted or unsubstituted lower alkyl, substituted or unsubstituted lower alkenyl or substituted or unsubstituted lower aralkyl and E1 represents a leaving group) to a nitrogen atom in Q.

(15) The quaternary ammonium salt of the pyrimidine derivative or the pharmaceutically acceptable salt thereof according to (14).

(16) A pharmaceutical composition which comprises, as an active ingredient, the pyrimidine derivative, or the quaternary ammonium salt thereof, or the pharmaceutically acceptable salt thereof according to any of (1) to (14).

(17) An anti-inflammatory agent which comprises, as an active ingredient, the pyrimidine derivative, or the quaternary ammonium salt thereof, or the pharmaceutically acceptable salt thereof according to any of (1) to (14).

(18) A therapeutic and/or preventive agent for a disease which is relared to thymus and activation-regulated chemokine (TARC) and/or macrophage-derived chemokine (MDC) which comprises, as an active ingredient, the pyrimidine derivative, or the quaternary ammonium salt thereof, or the pharmaceutically acceptable salt thereof according to any of (1) to (14).

(19) A pharmaceutical composition which comprises, as an active ingredient, the quaternary ammonium salt of the pyrimidine derivative or the pharmaceutically acceptable salt thereof according to (15).

(20) An anti-inflammatory agent which comprises, as an active ingredient, the quaternary ammonium salt of the pyrimidine derivative or the pharmaceutically acceptable salt thereof according to (15).

(21) A therapeutic and/or preventive agent for a disease which is relared to thymus and activation-regulated chemokine (TARC) and/or macrophage-derived chemokine (MDC) which comprises, as an active ingredient, the quaternary ammonium salt of the pyrimidine derivative or the pharmaceutically acceptable salt thereof according to (15).

(22) Use of the pyrimidine derivative, or the quaternary ammonium salt thereof, or the pharmaceutically acceptable salt thereof according to any of (1) to (14), for the manufacture of an anti-inflammatory agent.

(23) Use of the pyrimidine derivative, or the quaternary ammonium salt thereof, or the pharmaceutically acceptable salt thereof according to any of (1) to (14), for the manufacture of a therapeutic and/or preventive agent for a disease which is relared to thymus and activation-regulated chemokine (TARC) and/or macrophage-derived chemokine (MDC).

(24) Use of the quaternary ammonium salt of the pyrimidine derivative or the pharmaceutically acceptable salt thereof according to (15), for the manufacture of an anti-inflammatory agent.

(25) Use of the quaternary ammonium salt of the pyrimidine derivative or the pharmaceutically acceptable salt thereof according to (15), for the manufacture of a therapeutic and/or preventive agent for a disease which is relared to thymus and activation-regulated chemokine (TARC) and/or macrophage-derived chemokine (MDC).

(26) A method for treating and/or preventing inflammation, which comprises administering an effective amount of the pyrimidine derivative, or the quaternary ammonium salt thereof, or the pharmaceutically acceptable salt thereof according to any of (1) to (14).

(27) A method for treating and/or preventing a disease which is relared to thymus and activation-regulated chemokine (TARC) and/or macrophage-derived chemokine (MDC), which comprises administering an effective amount of the pyrimidine derivative, or the quaternary ammonium salt thereof, or the pharmaceutically acceptable salt thereof according to any of (1) to (14).

(28) A method for treating and/or preventing inflammation, which comprises administering an effective amount of the quaternary ammonium salt of the pyrimidine derivative or the pharmaceutically acceptable salt thereof according to (15).

(29) A method for treating and/or preventing a disease which is relared to thymus and activation-regulated chemokine (TARC) and/or macrophage-derived chemokine (MDC), which comprises administering an effective amount of the quaternary ammonium salt of the pyrimidine derivative or the pharmaceutically acceptable salt thereof according to (15).

A compound represented by Formula (I) is hereinafter referred to as Compound (I). The same shall apply to other compounds of other formula number.

In the definitions of the groups in Formulae (I) to (V),

Examples of the lower alkyl and the lower alkyl moiety of the lower alkoxy, the lower alkylthio, the lower alkylsulfinyl, the lower alkylsulfonyl, the lower alkoxylcarbonyl, the mono(lower alkyl)amino, the di(lower alkyl)amino, the mono(lower alkyl)aminocarbonyl, and the di(lower alkyl)aminocarbononyl include straight-chain or branched alkyl having 1 to 10 carbon atoms or cycloalkyl having 1 to 8 carbon atoms, such as methyl, ethyl, propyl, isopropyl, butyl, isobutyl, sec-butyl, tert-butyl, pentyl, neopentyl, hexyl, heptyl, octyl, isooctyl, nonyl, decyl, cyclopropyl, cyclobutyl, cyclopentyl, cyclohexyl, cycloheptyl or cyclooctyl. These examples further include combination of straight-chain or branched alkyl having 1 to 10 carbon atoms defined above and cycloalkyl having 1 to 8 carbon atoms defined above, such as cycloalkylalkyl or alkylcycloalkyl. Cycloalkylalkyl includes, for example, cyclopropylmethyl, cyclohexylmethyl, or cyclooctylmethyl. Alkylcycloalkyl includes, for example, methylcyclohexyl, ethylcyclohexyl, propylcyclohexyl, isopropylcyclohexyl, butylcyclohexyl, isobutylcyclohexyl, sec-butylcyclohexyl or tert-butylcyclohexyl. Two lower alkyl moieties of the di(lower alkyl)amino and the di(lower alkyl)aminocarbonyl may be the same or different.

Examples of the lower alkanoyl include straight-chain or branched alkanoyl having 1 to 7 carbon atoms, such as formyl, acetyl, propionyl, butyryl, isobutyryl, valeryl, isovaleryl, pivaloyl, hexanoyl or heptanoyl.

Examples of the lower alkenyl include straight-chain or branched alkenyl or alkadienyl having 2 to 8 carbon atoms, or cycloalkenyl having 4 to 10 carbon atoms, such as vinyl, allyl, methacryl, 1-propenyl, butenyl, crotyl, pentenyl, isoprenyl, hexenyl, heptenyl, octenyl, 2,6-octadienyl, cyclobutenyl, cyclopentenyl, cyclohexenyl, cycloheptenyl, cyclooctenyl, cyclononenyl or cyclodecenyl. These examples further include combination of straight-chain or branched alkenyl or alkadienyl having 2 to 8 carbon atoms defined above and cycloalkenyl having 4 to 10 carbon atoms defined above, such as 2-(1-cyclohexenyl)ethyl.

Examples of the lower alkynyl include alkynyl having 2 to 8 carbon atoms, such as ethynyl, propynyl, butynyl, pentynyl, hexynyl, heptynyl or octynyl.

Examples of the aryl and the aryl moiety of the aryloxy and the aroyl include monocyclic, bicyclic or tricyclic aryl having 6 to 14 carbon atoms, such as phenyl, naphthyl, indenyl or anthranyl. Examples of the aryl include residue of hydrocarbon ring assembly, such as biphenyl.

The aryl moiety of the aralkyl and the aralkyloxy include, in addition to the aryl defined above, condensed polycyclic groups wherein the aryl defined above and the cycloalkyl are condensed, such as indanyl, 1,2,3,4-tetrahydronaphthyl or 6,7,8,9-tetrahydro-5H-benzocycloheptyl.

The alkylene moiety of the aralkyl, the aralkyloxy, the heteroaromatic alkyl and the heteroalicyclic alkyl has the same meanings as the groups formed by removing one hydrogen atom from the straight-chain or branched alkyl having 1 to 10 carbon atoms defined above.

Examples of the heteroaromatic group and the heteroaromatic moiety of the heteroaromatic alkyl include 5-or 6-membered monocyclic heteroaromatic groups containing at least one atom selected from a nitrogen atom, an oxygen atom and a sulfur atom; and condensed bicyclic or tricyclic heteroaromatic groups in which 3- to 8-membered rings are condensed and containing at least one atom selected from a nitrogen atom, an oxygen atom and a sulfur atom. Specific examples thereof include furyl, thienyl, pyrrolyl, pyridyl, oxazolyl, thiazolyl, triazolyl, indolyl, quinolyl, purinyl, benzofuranyl, benzodioxolyl, benzoxazolyl, benzothiazolyl, benzimidazolyl and the like.

Examples of the heteroalicyclic group and the heteroalicyclic moiety of the heteroalicyclic alkyl include 5- or 6-membered monocyclic heteroalicyclic groups containing at least one atom selected from a nitrogen atom, an oxygen atom and a sulfur atom; a condensed bicyclic or tricyclic heteroalicyclic groups in which 3- to 8-membered rings are condensed and containing at least one selected from a nitrogen atom, an oxygen atom and a sulfur atom; and heteroalicyclic groups thereof in which an unsaturated bond(s) are also included. Specific examples thereof include tetrahydropyranyl, tetrahydrothiopyranyl, morpholino, thiomorpholino, morpholinyl, thiomorpholinyl, pyrrolidinyl, piperazinyl, homopiperazinyl, piperidino, homopiperidino, piperidinyl, homopiperidinyl, tetrahydrofuranyl, tetrahydroquinolyl, tetrahydroisoquinolyl, octahydroquinolyl, dihydrobenzofuranyl, tetrahydropyridyl, 1,3-dioxolanyl, perhydroazepinyl, perhydroazocinyl and the like.

Examples of the heteroalicyclic group formed together with the adjacent nitrogen atom include 5- or 6-membered monocyclic heteroalicyclic groups containing at least one nitrogen atom (the monocyclic heterocyclic groups may contain any of other nitrogen atom, an oxygen atom and a sulfur atom); and condensed bicyclic or tricyclic heterocyclic groups in which 3- to 8-membered rings are condensed and containing at least one nitrogen atom (the condensed heterocyclic group may contain any of other nitrogen atom, an oxygen atom and a sulfur atom); heterocyclic groups thereof in which an unsaturated bond(s) are also included. Specific examples thereof include piperidino, homopiperidino, piperazinyl, homopiperazinyl, morpholino, thiomorpholino, perhydroazepinyl, perhydroazocinyl, tetrahydroquinolyl, tetrahydroisoquinolyl, octahydroquinolyl, tetrahydropyridyl and the like.

Examples of the substituents of the substituted lower alkyl, which may be the same or different and in number of 1 to 3, include hydroxy, amino, azido, carboxy, carbamoyl, nitro, cyano, mercapto, carbamoyl, halogen, lower alkoxy, mono(lower alkyl)amino, di(lower alkyl)amino, lower alkylthio, lower alkoxycarbonyl, lower alkanoyl, halogenated lower alkanoyl, aryloxy, aralkyloxy, aroyl, mono(lower alkyl)aminocarbonyl, di(lower alkyl)aminocarbonyl, substituted or unsubstituted arylsulfonyloxy (wherein the substituent of the substituted arylsulfonyloxy include a lower alkyl and the like), lower alkylsulfinyl, lower alkylsulfonyl, lower alkylsulfonyloxy, halogenated lower alkylsulfonyloxy and the like.

Here, the lower alkyl, the lower alkoxy, the mono(lower alkyl)amino, the di(lower alkyl)amino, the lower alkylthio, the lower alkoxycarbonyl, the lower alkanoyl, the aryloxy, the aralkyloxy, the aroyl, the mono(lower alkyl)aminocarbonyl, the di(lower alkyl)aminocarbonyl, the lower alkylsulfinyl, and the lower alkylsulfonyl have the same meanings as defined above, respectively. The halogen means a fluorine atom, a chlorine atom, a bromine atom or an iodine atom. The halogen moieties of the halogenated lower alkylsulfonyloxy and the halogenated lower alkanoyl, which may be the same or different and in number of 1 to 3, have the same meanings as defined above, respectively. The lower alkyl moiety of the lower alkylsulfonyloxy has the same meaning as the lower alkyl defined above. The lower alkylene moiety of the halogenated lower alkylsulfonyloxy has the same meaning as the group formed by removing one hydrogen atom from the straight-chain or branched alkyl having 1 to 10 carbon atom(s) cited in the definition of the lower alkyl defined above. The lower alkanoyl moiety of the halogenated lower alkanoyl moiety has the same meaning as the group formed by removing one hydrogen atom from the lower alkanoyl defined above. The aryl moiety of the arylsulfonyloxy has the same meaning as the aryl defined above.

Examples of the substituents of the substituted lower alkenyl, the substituted lower alkynyl, the substituted lower alkanoyl, the substituted lower alkoxy, the substituted lower alkoxycarbonyl, the substituted mono(lower alkyl) amino, the substituted di(lower alkyl)amino, the substituted mono(lower alkyl)aminocarbonyl, the substituted di(lower

alkyl)aminocarbonyl, the substituted lower alkylsulfinyl and the substituted lower alkylsulfonyl, which may be the same or different and in number of 1 to 3, include a heteroaromatic group and a heteroalicyclic group, in addition to the groups listed in the definition of the substituents of the substituted lower alkyl.

Here, the heteroaromatic group and the heteroalicyclic group have the same meanings as the heteroaromatic group and heteroalicyclic group defined above, respectively.

Examples of the substituents of the substituted mono(lower alkyl)amino, the substituted di(lower alkyl)amino and the substituted alkoxy, which may be the same or different and in number of 1 to 3, include a heteroalicyclic group in addition to the groups listed in the definition of the substituents of the substituted lower alkyl.

Here, the heteroalicyclic group has the same meaning as the heteroalicyclic group defined above.

Examples of the substituents of the substituted aryl, the substituted aralkyl, the substituted heteroaromatic group, the substituted heteroalicyclic group, the substituted heteroaromatic alkyl, the substituted heteroalicyclic alkyl, the substituted heteroalicyclic group formed together with the adjacent nitrogen atom, the substituted aryloxy, the substituted aralkyloxy, the substituted aroyl, the substituted phenyl, the substituted naphthyl, the substituted biphenyl, the substituted thienyl, the substituted thiazolyl, the substituted benzofuranyl, the substituted furyl, the substituted pyridyl, the substituted perhydroazepin-4-ylamino, the substituted piperidin-4-ylamino, the substituted 1,2,5,6-tetrahydropyridin-1-yl, the substituted 1,2,3,4-tetrahydroisoquinolin-2-yl and the substituted piperidino, which may be the same or different and in number of 1 to 3, include hydroxyl, nitro, cyano, carboxy, carbamoyl, methylenedioxy, amino, mercapto, halogen, lower alkyl moiety, substituted lower alkyl (wherein the substituent of the substituted lower alkyl has the same meaning as the substituent of the substituted lower alkyl defined above), lower alkoxy, substituted lower alkoxy (wherein the substituent of the substituted lower alkoxy has the same meaning as the substituent of the substituted lower alkoxy defined above), lower alkylthio, lower alkenyl, substituted lower alkenyl (wherein the substituent of the substituted lower alkenyl has the same meaning as the substituent of the substituted lower alkenyl defined above), lower alkynyl, substituted lower alkynyl (wherein the substituent of the substituted lower alkynyl has the same meaning as the substituent of the substituted lower alkynyl defined above), lower alkoxycarbonyl, substituted lower alkoxycarbonyl (wherein the substituent of the substituted lower alkoxycarbonyl has the same meaning as the substituent of the substituted lower alkoxycarbonyl defined above), lower alkanoyl, substituted lower alkanoyl (wherein the substituent of the substituted lower alkanoyl has the same meaning as the substituent of the substituted lower alkanoyl defined above), aryl, substituted aryl (wherein the substituent of the substituted aryl, which may be the same or different and in number of 1 to 3, include halogen, hydroxy and the like), aralkyl, substituted aralkyl (wherein the substituent of the substituted aralkyl, which may be the same or different and in number of 1 to 3, include halogen, hydroxy and the like), aryloxy, substituted aryloxy (wherein the substituent of the substituted aryloxy, which may be the same or different and in number of 1 to 3, include halogen, hydroxy and the like), aralkyloxy, substituted aralkyloxy (wherein the substituent of the substituted aralkyloxy, which may be the same or different and in number of 1 to 3, include halogen, hydroxy and the like), aroyl, substituted aroyl (wherein the substituent of the substituted aroyl, which may be the same or different and in number of 1 to 3, include halogen, hydroxy and the like) a heteroalicyclic group, a heteroaromatic group, mono(lower alkyl) amino, substituted mono(lower alkyl)amino [wherein the substituent of the substituted mono(lower alkyl)amino has the same meaning as the substituent of the substituted mono(lower alkyl)amino defined above], di(lower alkyl)amino, substituted di(lower alkyl)amino [wherein the substituent of the substituted di(lower alkyl)amino has the same meaning as the substituent of the substituted di(lower alkyl)amino defined above], mono(lower alkyl)aminocarbonyl, substituted mono(lower alkyl)aminocarbonyl moiety [wherein the substituent of the substituted mono(lower alkyl)aminocarbonyl has the same meaning as the substituent of the substituted mono(lower alkyl)aminocarbonyl defined above], di(lower alkyl)aminocarbonyl, substituted di(lower alkyl)aminocarbonyl [wherein the substituent of the substituted di(lower alkyl) aminocarbonyl has the same meaning as the substituent of the substituted di(lower alkyl)aminocarbonyl defined above], lower alkylsulfinyl, substituted lower alkylsulfinyl [wherein the substituent of the substituted lower alkylsulfinyl has the same meaning as the substituent of the substituted lower alkylsulfinyl defined above], lower alkylsulfonyl, substituted lower alkylsulfonyl [wherein the substituent of the substituted lower alkylsulfonyl has the same meaning as the substituent of the substituted lower alkylsulfonyl defined above] and the like. Examples of the substituents of the alkylene moiety of the substituted aralkyl and the heteroaromatic alkyl, which may be the same or different and in number of 1 or 2, include aryl and the like.

Here, the halogen, the lower alkyl, the lower alkoxy, the lower alkylthio, the lower alkenyl, the lower alkynyl, the lower alkoxycarbonyl, the lower alkanoyl, the halogenated lower alkanoyl, the aryl, the aralkyl, the aryloxy, the aralkyloxy, the aroyl, the heteroalicyclic group, the heteroaromatic group, the mono(lower alkyl)amino, the di(lower alkyl)amino, the mono(lower alkyl)aminocarbonyl, the di(lower alkyl)aminocarbonyl, the lower alkylsulfinyl and the lower alkylsulfonyl have the same meanings as defined above, respectively.

Examples of the leaving group include halogen, substituted or unsubstituted arylsulfonyloxy (wherein the substituent of the substituted arylsulfonyloxy include lower alkyl and the like) and substituted or unsubstituted lower alkylsulfonyloxy (wherein the substituent of the substituted alkylsulfonyloxy include halogen and the like) and the like.

Here, the halogen, the aryl moiety of the arylsulfonyloxy, lower alkyl and the lower alkyl moiety of the lower

alkylsulfonyloxy have the same as the halogen, the aryl and the lower alkyl defined above, respectively.

The substitutable number represents a structural maximum number of the substituent that can be substituted. In particular, a, $a^1$, $a^2$ and $a^3$ preferably represent an integer of 0 to 6 and more preferably 0 to 3, respectively.

The quaternary ammonium salts of Compound (I) may be any quaternary ammonium salts formed by the addition of, for example, Z-El (wherein Z and E1 have the same meaning as defined above, respectively) to, for example, 1 to 3 nitrogen atom(s) in the structure of Compound (I). Specific examples of the quaternary ammonium salts include quaternary ammonium salts [$-N^+(Z)El^-$- (wherein Z and E1 have the same meanings as defined above, respectively)] each formed by the addition of Z-E1 (wherein Z and E1 have the same meanings as defined above, respectively) to any nitrogen atom in Q of Compound (I). Z is preferably lower alkyl or lower alkenyl and more preferably methyl, ethyl or allyl. E1 is preferably halogen and more preferably an iodine atom or a bromine atom.

The pharmaceutically acceptable salts of Compound (I) are preferably nontoxic and water-soluble. Examples of the pharmaceutically acceptable salts include acid addition salts including inorganic acid salts such as hydrochlorides, hydrobromides, nitrates, sulfates, phosphates and the like, and organic acid salts such as actates, maleates, citrates, fumarates and the like; alkali metal salts such as sodium salts and potassium salts; alkaline earth metal salts such as magnesium salts and calcium salts; metal salts such as aluminum salts and zinc salts; ammonium salts such as ammonium and tetramethylammonium; organic amine addition salts such as morpholine addition salts and piperidine addition salts; and amino acid addition salts such as glycine addition salts, phenylalanine addition salts, lysine addition salts, aspartic acid addition salts, and glutamic acid addition salts.

The present invention includes any compounds, derived from Compound (I), labeled with one or more isotopes, a fluorescent compound, biotin, an enzyme, or the like. Such labeled compounds are useful for investigating the distribution of a compound in tissues and researching compounds having an activity of regulating functions of TARC and/or MDC.

The isotope means an atom having a valence or mass different from that of a naturally-occurring atom. Examples of an isotope used herein include $^{2}H$, $^{3}H$, $^{13}C$, $^{14}C$, $^{15}N$, $^{17}O$, $^{18}O$, $^{31}P$, $^{32}P$, $^{35}S$, $^{36}Cl$ and the like.

Production methods of Compound (I) will now be described.

When a defined group changes under the reaction conditions or is not suitable for carrying out the methods in the following production methods, it is possible to carry out the production using a method for introduction and elimination of protective group commonly used in synthetic organic chemistry [for example, *Protective Groups in Organic Synthesis,* third edition, T. W. Greene, John Wiley & Sons Inc]. If necessary, the order of reaction processes such as introduction of substituents can be changed.

Compound (I) can be obtained according to, for example, Production methods 1 to 5 described below.

Production method 1:

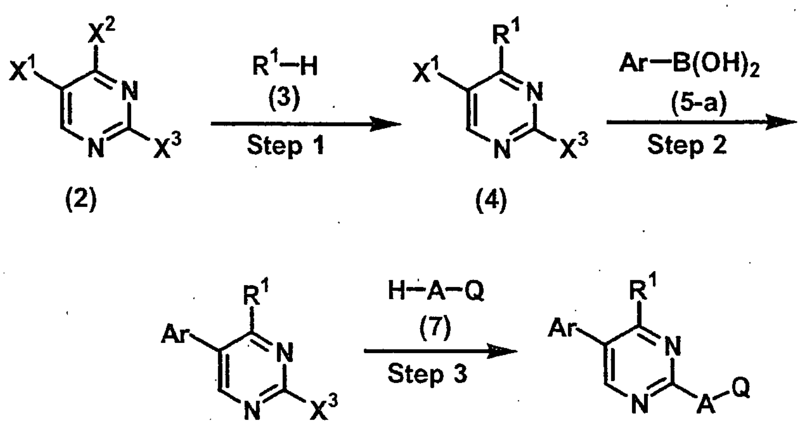

(2) (4) (6) (I)

(wherein $R^1$, Ar, A and Q have the same meanings as defined above, respectively and $X^1$, $X^2$ and $X^3$ may be the same or different and each represents a chlorine atom, a bromine atom or an iodine atom)

[Step 1]

Compound (4) can be obtained by reacting Compound (2) with 1 equivalent to a large excess of, preferably

1 to 3 equivalents of Compound (3) in a solvent inert to the reaction. The reaction is performed at a temperature of 0°C to the boiling point of the solvent, preferably at 50 to 120°C, usually for 10 minutes to 48 hours.

Compound (2) is commercially available or can be synthesized according to a procedure disclosed in a literature [for example, *Org. Prep. Proced. Int.,* vol. 30, pp. 433-437 (1998)].

Compound (3) is commercially available or can be synthesized according to a procedure disclosed in a literature [for example, *Shin Jikken Kagaku Koza 14,* "Synthesis and Reaction of Organic Compound (III)", Maruzen, pp. 1332-1399 (1977); *Jikken Kagaku Koza* 20, "Organic Synthesis II (Alcohol and Amine)", Maruzen, pp. 279-318 (2001)].

Any solvent can be used so long as it is inert to the reaction. Examples of the solvent include tetrahydrofuran (THF), dioxane, diethyl ether, diisopropyl ether, benzene, toluene, xylene, ethyl acetate, acetonitrile, dichloromethane, chloroform, dichloroethane, dimethylformamide (DMF), dimethylacetoamide (DMA), N-methylpyrrolidone, dimethyl sulfoxide (DMSO), methanol, ethanol and the like. These solvents may be used alone or in combination. Among them, THF, chloroform, ethanol or a mixture thereof is preferably used.

1 equivalent to a large excess of, preferably 1 to 10 equivalents of base may be used in the reaction.

Examples of the base include organic bases such as triethylamine, diisopropylethylamine, 1,8-diazabicyclo [5.4.0]undec-7-ene (DBU), N,N-dimethylaniline, pyridine and quinoline; inorganic bases such as potassium carbonate, sodium carbonate, lithium carbonate, sodium hydrogencarbonate, potassium hydroxide, sodium hydroxide, lithium hydroxide and potassium tert-butoxide; basic anion-exchange resins such as AMBERLYST A-21 (available from Rhom and Haas Company) and AG1-X8 (available from Bio-Rad Laboratories, Inc.); base immobilized on a solid such as polyvinyl pyridine and morpholinomethyl polystyrene, and the like. Among them, Triethylamine is preferably used.

[Step 2]

Compound (6) can be obtained by reacting Compound (4) obtained in Step 1 with 1 to 5 equivalents of boronic acid derivative (5-a) in the presence of 1 equivalent to a large excess of, preferably 1 to 3 equivalents of base and 0.05 to 1 equivalent of, preferably 0.05 to 0.10 equivalent of palladium catalyst, in a solvent inert to the reaction. The reaction is performed at a temperature of 0 to 150°C, preferably at 20 to 120°C, usually for 1 to 72 hours.

The boronic acid derivative (5-a) is commercially available or can be synthesized according to a procedure disclosed in a literature [for example, *Chem. Rev*., vol. 95, p. 2457 (1995); Tetrahedron Lett., vol. 38, p. 1197 (1995); *J. Org. Chem*., vol. 60, p. 7508 (1995); or *J. Org. Chem.,* vol. 65, p. 9268 (2000)].

Any solvent can be used so long as it is inert to the reaction. Examples of the solvent include THF, dioxane, diethyl ether, diisopropyl ether, dimethoxyethane, DMF, DMA, N-methylpyrrolidone, DMSO, benzene, toluene, xylene, ethyl acetate, acetonitrile, hexamethylphosphoramide (HMPA), acetone, ethanol, methanol, triethylamine, 1,3-dimethyl-3,4,5,6-tetrahydro-2(1H)-pyrimidinone (DMPU), water and the like. These solvents may be used alone or in combination. Among them, dimethoxyethane or toluene is preferably used.

Examples of the palladium catalyst include palladium(II) acetate, palladium(II) chloride, dichlorobis(triphenylphosphine)palladium(II), bis(acetonitrile)dichloropalladium(II), bis(benzonitrile)dichloropalladium(II), [1,2-bis(diphenylphosphino)ethane]dichloropalladium(II), [1,1'-bis(diphenylphosphino)ferrocene]palladium(II) dichloride, [2,2'-bis (diphenylphosphino)-1,1'-binaphthyl]palladium(II) chloride, dichlorobis(tricyclohexylphosphine)palladium(II), dichlorobis(tri-o-tolylphosphine)palladium(II), [1,4-bis(diphenylphosphino)butane]palladium(II) dichloride, bis(acetate)bis (triphenylphosphine)palladium(II), palladium(II) trifluoroacetate, tetrakis(triphenylphosphine)palladium(0), tris(dibenzylideneacetone)dipalladium(0), palladium(0)/carbon (Pd/C) and the like. The organophosphorus compound may be used in combination with the palladium catalyst. Examples of the organophosphorus compound include triphenylphosphine, o-tolylphosphine, tri-tert-butylphosphine, tri-2-furylphosphine, 1,2-bis(diphenylphosphino)ethane, 1,1'-bis (diphenylphosphino)ferrocene, 2,2'-bis(diphenylphosphino)-1,1'-binaphthyl, tricyclohexylphosphine, 1,2-bis(diphenylphosphino)ethane, 1,3-bis(diphenylphosphino)propane, 1,4-bis(diphenylphosphino)butane and the like. Among them, tetrakis(triphenylphosphine)palladium(0), dichlorobis(triphenylphosphine)palladium(II), dichlorobis(tri-o-tolylphosphine)palladium(II) or [1,1'-bis(diphenylphosphino)ferrocene] palladium(II) dichloride is preferably used.

Examples of the base include organic bases such as triethylamine, diisopropylethylamine, DBU, N,N-dimethylaniline, pyridine and quinoline; inorganic bases such as cesium carbonate, potassium carbonate, sodium carbonate, lithium carbonate, sodium hydrogencarbonate, potassium hydroxide, sodium hydroxide, lithium hydroxide, potassium tert-butoxide, thallium hydroxide, thallium carbonate, silver carbonate and silver oxide; basic anion-exchange resins such as AMBERLYST A-21 (available from Rhom and Haas Company) and AG1-X8 (available from Bio-Rad Laboratories, Inc.); base immobilized on a solid such as polyvinylpyridine and morpholinomethyl polystyrene, and the like. Among them, cesium carbonate is preferably used.

[Step 3]

Compound (I) can be obtained by reacting Compound (6) obtained in Step 2 with 1 to 5 equivalents of Compound (7) or a salt thereof in the presence of 1 equivalent to a large excess of, preferably 20 to 25 equivalents of base, in a solvent inert to the reaction. The reaction is performed at a temperature of 0 to 150°C, preferably at 20 to 130°C, usually for 1 to 72 hours.

Examples of the salt of Compound (7) include hydrochlorides, sulfates and the like. Compound (7) or the salt is commercially available or can be synthesized according to a procedure disclosed in a literature [for example, *J. Org. Chem.,* vol. 63, p. 4131 (1998); *J. Org.* Chem., vol. 55, p. 2552 (1990)].

Any solvent can be used so long as it is inert to the reaction. Examples of the solvent include dichloromethane, chloroform, dichloroethane, DMF, DMA, N-methylpyrrolidone, DMSO, THF, dioxane, diethyl ether, diisopropyl ether, benzene, toluene, xylene, ethyl acetate, acetonitrile and the like. These solvents may be used alone or in combination. Among them, DMF is preferably used.

Examples of the base include organic bases such as triethylamine, pyridine, N,N-dimethylaniline, N,N-diethylaniline, diisopropylethylamine, 2,2,6,6-tetramethylpiperidine, 1,4-diazabicyclo[2.2.2]octane and DBU; inorganic bases such as cesium carbonate, potassium carbonate, sodium carbonate, lithium carbonate, sodium hydrogencarbonate, potassium hydroxide, sodium hydroxide, lithium hydroxide, potassium tert-butoxide, thallium hydroxide, thallium carbonate, silver carbonate and silver oxide; basic anion-exchange resins such as AMBERLYST A-21 (available from Rhom and Haas Company) and AG1-X8 (available from Bio-Rad Laboratories, Inc.); base immobilized on a solid such as polyvinylpyridine and morpholinomethyl polystyrene, and the like. Among them, triethylamine is preferably used.

Furthermore, Compound (I) can be obtained by Production method 2 described below.

Production method 2:

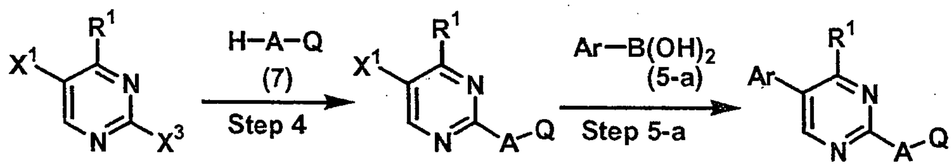


(4) (8) (I)

(wherein $R^1$, Ar, A, Q, $X^1$ and $X^2$ have the same meanings as defined above, respectively)

[Step 4]

Compound (8) can be obtained by reacting Compound (4) obtained in Step 1 with Compound (7) or a salt thereof in a similar manner to Step 3.

[Step 5-a]

Compound (I) can be obtained by reacting Compound (8) obtained in Step 4 with boronic acid derivative (5-a) in a similar manner to Step 2.

Furthermore, Compound (I) can be obtained according to Production method 3 described below.

Production method 3:

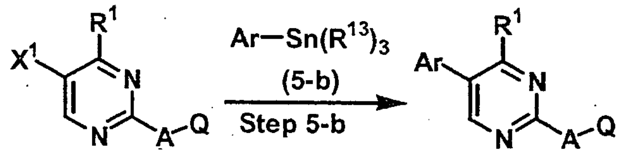


(8) (I)

[wherein $R^1$, Ar, A, Q and $X^1$ have the same meanings as defined above, respectively and three $R^{13}$ are the same and represent lower alkyl (the lower alkyl has the same meaning as defined above)]

[Step 5-b]

Compound (I) can be obtained by reacting Compound (8) obtained in Step 4 with 1 to 5 equivalents of organotin compound (5-b) in the presence of 1 equivalent to a large excess of, preferably 0.05 to 0.10 equivalents of palladium catalyst, in a solvent inert to this reaction. The reaction is performed at a temperature of 0 to 100°C, preferably at 20 to 90°C, usually for 1 to 72 hours.

The organotin compound (5-b) is commercially available or can be synthesized according to a procedure disclosed in a literature [for example, *Jikken Kagaku Koza* 24, "Organic Synthesis VI (Heteroelements and Typical Metal Compounds)", Maruzen, pp. 179-201 (1992); Angew. Chem. Int. Ed. Engl., vol. 25, pp. 508-521 (1986)].

Any solvent can be used so long as it is inert to the reaction. Examples of the solvent include THF, dioxane, diethyl ether, diisopropyl ether, dimethoxyethane, DMF, DMA, N-methylpyrrolidone, DMSO, benzene, toluene, xylene, ethyl acetate, acetonitrile, HMPA, acetone, ethanol, methanol, triethylamine, DMPU, water and the like. These solvents may be used alone or in combination. Among them, THF is preferably used.

The palladium catalyst may be the same as that cited in Step 2. The organophosphorus compound cited in Step 2 may be used in combination with the palladium catalyst. Among them, tetrakis(triphenylphosphine)palladium (0) or dichlorobis(triphenylphosphine)palladium(II) is preferably used.

1 equivalent to a large excess of, preferably 1 to 10 equivalents of fluoride may be used in the reaction.

Examples of the fluoride include tetra-n-butyl ammonium chloride, lithium fluoride, sodium fluoride, potassium fluoride, rubidium fluoride, cesium fluoride and the like. Among them, cesium fluoride or potassium fluoride is preferably used.

1 equivalent to a large excess of, preferably 1 to 10 equivalents of copper or silver salt may be used in the reaction.

Examples of the copper salt include copper oxide, copper chloride, copper bromide, copper iodide, copper cyanide and the like. Examples of the silver salt include silver oxide and the like.

Furthermore, Compound (I) can be obtained by Production method 4 described below.

Production method 4:

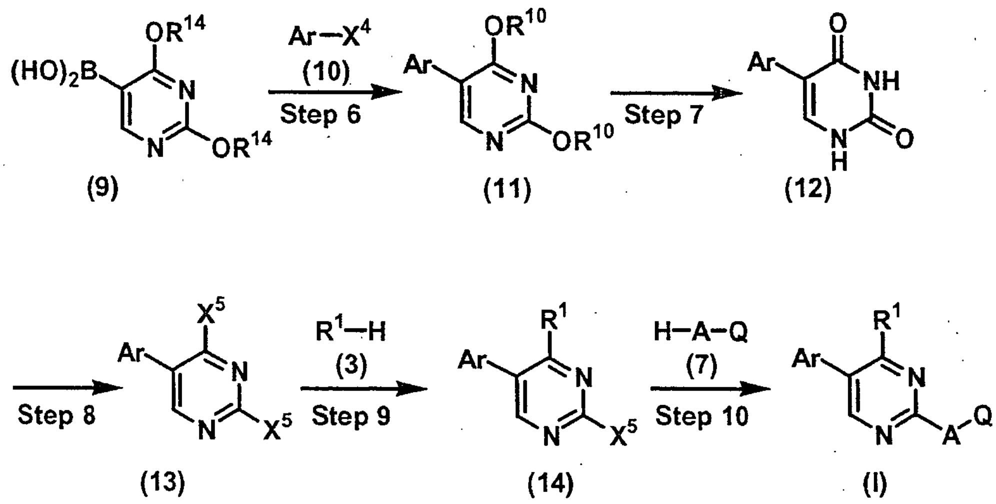

(9) (11) (12) (13) (14) (I)

[wherein $R^1$, Ar, A and Q have the same meanings as defined above, respectively, $X^4$ and $X^5$ may be the same or different and each represents a chlorine atom, a bromine atom or an iodine atom and $R^{14}$ represents substituted or unsubstituted lower alkyl (the lower alkyl has the same meaning as the lower alkyl defined above and the substituent (s) of the substituted alkyl are, for example, halogen which has the same meaning as the halogen defined above, and the like) or substituted or unsubstituted aralkyl (the aralkyl has the same meaning as the aralkyl defined above, the substituent(s) of the substituted aralkyl are, for example, halogen, lower alkyl, lower alkoxy and the like, and the halogen, the lower alkyl and the lower alkoxy have the same meanings as the halogen, the lower alkyl and the lower alkoxy

defined above, respectively)]

[Step 6]

Compound (11) can be obtained by reacting Compound (9) with 1 to 5 equivalents of Compound (10) in the presence of 1 equivalent to a large excess of, preferably 1 to 3 equivalents of base and 0.05 to 1.00 equivalent of, preferably 0.05 to 0.10 equivalent of palladium catalyst, in a solvent inert to this reaction. The reaction is performed at a temperature of 0 to 150°C, preferably at 20 to 120°C, usually for 1 to 72 hours.

Compound (9) can be synthesized according to a procedure disclosed in a literature [for example, *J. Chem. Soc*., p. 1051 (1968)]. Compound (9) wherein $R^{14}$ is tert-butyl is preferably used.

Compound (10) is commercially available or can be synthesized according to a procedure disclosed in a literature *[Shin Jikken Kagaku Koza* 14, "Synthesis and Reaction of Organic Compound (I)", Maruzen, (1978); *Jikken Kagaku Koza* 19, "Organic Synthesis I (Hydrocarbon and Halide)", Maruzen, pp. 416-479 (1992)].

Any solvent can be used so long as it is inert to the reaction. Examples of the solvent include THF, dioxane, diethyl ether, diisopropyl ether, dimethoxyethane, DMF, DMA, N-methylpyrrolidone, DMSO, benzene, toluene, xylene, ethyl acetate, acetonitrile, HMPA, acetone, ethanol, methanol, triethylamine, DMPU, water and the like. These solvents may be used alone or in combination. Among them, dimethoxyethane or toluene is preferably used.

The palladium catalyst may be the same as that cited in Step 2. The organophosphorus compound cited in Step 2 may be used in combination with the palladium catalyst. Among them, tetrakis(triphenylphosphine)palladium (0) or dichlorobis(triphenylphosphine)palladium(II) is preferably used.

Examples of the base include organic bases such as triethylamine, diisopropylethylamine, DBU, N,N-dimethylaniline, pyridine and quinoline; inorganic bases such as cesium carbonate, potassium carbonate, sodium carbonate, lithium carbonate, sodium hydrogencarbonate, potassium hydroxide, sodium hydroxide, lithium hydroxide, potassium tert-butoxide, thallium hydroxide, thallium carbonate, silver carbonate and silver oxide; basic anion-exchange resins such as AMBERLYST A-21 (available from Rhom and Haas Company) and AG1-X8 (available from Bio-Rad Laboratories, Inc.); base immobilized on a solid such as polyvinylpyridine and morpholinomethyl polystyrene, and the like. Among them, cesium carbonate is preferably used.

[Step 7]

Compound (12) can be obtained by treating Compound (11) obtained in Step 6 with 2 equivalents to a large excess of water in the presence of 1 to 10 equivalents of acid, in a solvent inert to the reaction. The reaction is performed at a temperature of 0 to 100°C, preferably at 20 to 50°C, usually for 1 to 5 hours.

Examples of the acid include carboxylic acids such as trifluoroacetic acid, mineral acids such as hydrochloric acid, and sulfonic acids such as trifluoromethane sulfonic acid and benzene sulfonic acid, etc. Among them, hydrochloric acid is preferably used.

Any solvent can be used so long as it is inert to the reaction. Examples of the solvent include pentane, hexane, THF, dioxane, diethyl ether, diisopropyl ether, dimethoxyethane, DMSO, benzene, toluene, xylene, HMPA, ethanol, methanol, water and the like. These solvents may be used alone or in combination. Among them, methanol is preferably used.

[Step 8]

Compound (13) can be obtained by treating Compound (12) obtained in Step 7 with 1 equivalent to an excess of, preferably an excess of halogenating agent in the presence or absence of 1 equivalent to a large excess of, preferably 1 to 3 equivalents of base, in a solvent inert to the reaction or without solvent. The reaction is performed at a temperature of 0°C to the boiling point of the solvent, preferably at 50°C to the boiling point of the solvent, usually for 1 to 48 hours.

Examples of the base include organic bases such as triethylamine, diisopropylethylamine, DBU, N,N-dimethylaniline, pyridine and quinoline; inorganic bases such as cesium carbonate, potassium carbonate, sodium carbonate, lithium carbonate, sodium hydrogencarbonate, potassium hydroxide, sodium hydroxide, lithium hydroxide and potassium tert-butoxide; basic anion-exchange resins such as AMBERLYST A-21 (available from Rhom and Haas Company) and AG1-X8 (available from Bio-Rad Laboratories, Inc.); base immobilized on a solid such as polyvinylpyridine and morpholinomethyl polystyrene, and the like. Among them, diisopropylethylamine is preferably used.

Examples of the halogenating agent include phosphorus trichloride, phosphorus tribromide, phosphorus pentachloride, phosphorus pentabromide, phosphorus oxychloride, phosphorus oxybromide and the like. These phosphorus compounds may be used alone or in combination. Among them, phosphorus oxychloride is preferably used.

Any solvent can be used so long as it is inert to the reaction. Examples of the solvent include THF, dioxane, chloroform, dichloroethane, benzene, toluene, xylene, ethyl acetate, triethylamine, pyridine, N,N-dimethylaniline, diisopropylethylamine and the like. These solvents may be used alone or in combination.

[Step 9]

Compound (14) can be obtained by reacting Compound (13) obtained in Step 8 with Compound (3) in a similar manner to Step 1.

[Step 10]

Compound (I) can be obtained by reacting Compound (14) obtained in Step 9 with Compound (7) or a salt thereof in a similar manner to Step 3.
Furthermore, Compound (I) can be obtained according to Production method 5 described below.

Production method 5:

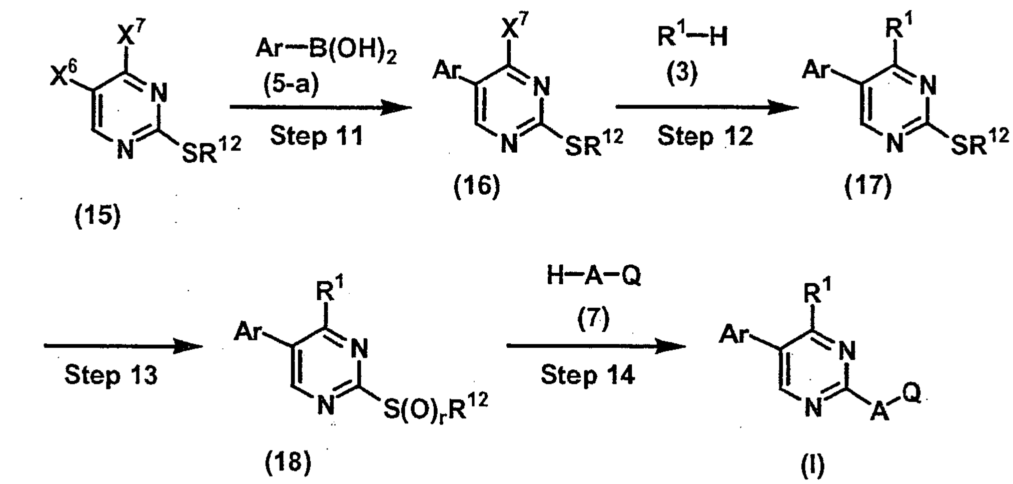


[wherein $R^1$, Ar, A and Q have the same meanings as defined above, respectively, $X^6$ and $X^7$ may be the same or different and each represents a chlorine atom, a bromine atom or an iodine atom, $R^{12}$ represents substituted or unsubstituted lower alkyl (the lower alkyl has the same meaning as lower alkyl defined above and the substituent(s) of the substituted alkyl are, for example, halogen which has the same meaning as the halogen defined above, and the like) and r represents 1 or 2]

[Step 11]

Compound (16) can be obtained by reacting Compound (15) with boronic acid derivative (5-a) in a similar manner to Step 2.
Compound (15) is commercially available or can be synthesized according to a procedure disclosed in a literature [for example, *Acta Chem. Scand.,* Ser B., vol. B36 (1), pp. 15-18 (1982)].

[Step 12]

Compound (17) can be obtained by reacting Compound (16) obtained in Step 11 with Compound (3) in a similar manner to Step 1.
A solvent and base used in the reaction may be the same as those cited in Step 1. Among them, dioxane and diisopropylethylamine are preferably used.

[Step 13]

Compound (18) can be obtained by reacting Compound (17) obtained in Step 12 with 1 equivalent to a large excess of, preferably 1 to 5 equivalents of oxidizing agent in a solvent inert to the reaction. The reaction is performed at a temperature of 0 to 100°C, preferably at 0 to 50°C, usually for 10 minutes to 24 hours.
Any solvent can be used so long as it is inert to the reaction. Examples of the solvent include dichloromethane, chloroform, dichloroethane, THF, dioxane, diethyl ether, diisopropyl ether, benzene, toluene, xylene, ethyl acetate, acetonitrile, water and the like. These solvents may be used alone or in combination. Among them, dichloromethane or toluene is preferably used.
Examples of the oxidizing agent include 3-chloroperbenzoic acid (mCPBA), benzoyl peroxide, peracetic acid, aqueous hydrogen peroxide and the like. Among them, mCPBA is preferably used.
In Compound (18), each of compounds wherein r is 1 and compounds wherein r is 2 can separately be obtained by adjusting the reaction conditions such as equivalents of the oxidizing agent, the reaction temperature, etc., but both of the compounds may be obtained as a mixture thereof. In case where the mixture is obtained, the ratio of the two compounds is not particularly limited, and mixtures having any ratios can be used as such in a subsequent step.

[Step 14]

Compound (I) can be obtained by reacting Compound (18) obtained in Step 13 with 1 to 5 equivalents of Compound (7) or a salt thereof in a solvent inert to the reaction. The reaction is performed at a temperature of 0°C to the boiling point of the solvent, preferably at 25 to 120°C, usually for 1 to 72 hours.
Any solvent can be used so long as it is inert to the reaction. Examples of the solvent include dichloromethane, chloroform, dichloroethane, DMF, DMA, N-methylpyrrolidone, DMSO, THF, dioxane, diethyl ether, diisopropyl ether, benzene, toluene, xylene, ethyl acetate, acetonitrile, ethanol and the like. These solvents may be used alone or in combination. Among them, dioxane or ethanol is preferably used.
1 equivalent to a large excess of, preferably 1 to 10 equivalents of base may be used in the reaction.
Examples of the base include organic bases such as triethylamine, diisopropylethylamine, DBU, N,N-dimethylaniline, pyridine and quinoline; inorganic bases such as potassium carbonate, sodium carbonate, lithium carbonate, sodium hydrogencarbonate, potassium hydroxide, sodium hydroxide, lithium hydroxide and potassium tert-butoxide; basic anion-exchange resins such as AMBERLYST A-21 (available from Rhom and Haas Company) and AG1-X8 (available from Bio-Rad Laboratories, Inc.); base immobilized on a solid such as polyvinylpyridine and morpholinomethyl polystyrene, and the like. Among them, diisopropylethylamine is preferably used.

Production method 6

A quaternary ammonium salt of Compound (I) can be prepared according to, for example, process described below.
The desired quaternary ammonium salt of Compound (I) can be obtained by reacting Compound (I) obtained in Production methods 1 to 5 with 1 equivalent to a large excess of, preferably 1 to 20 equivalents of Z-E1 (wherein Z and E1 have the same meanings as defined above, respectively) in a solvent inert to the reaction or without a solvent.
Any solvent can be used so long as it is inert to the reaction. Examples of the solvent include THF, dioxane, diethyl ether, diisopropyl ether, benzene, toluene, xylene, ethyl acetate, acetonitrile, methanol, ethanol, 1-propanol, 2-propanol, 1-butanol, 2-butanol, dichloromethane, chloroform, dichloroethane, DMF, DMA, N-methylpyrrolidone, DMSO, nitromethane, acetone, methyl ethyl ketone and the like. These solvents may be used alone or in combination. Among them, dichloromethane or nitromethane is preferably used.
The reaction is performed at a temperature of -20°C to the boiling point of the solvent or Z-El, preferably at 0 to 30°C, usually for 1 hour to 1 week.
The conversion of functional groups of Compound (I), starting materials and intermediates, and functional groups in the substituents thereof can be carried out according to a known procedure [for example, *Comprehensive Organic Transformations,* second edition, R. C. Larock, published by John Wiley & Sons Inc., (1999)].
Compound (I) having desired functional groups at desired positions can be obtained according to a suitable combination of the methods described above and the like.
The products and intermediates prepared in the Production methods described above can be isolated or purified according to any suitable combination of techniques generally used in organic syntheses such as filtration, extraction, washing, drying, concentration, crystallization, various chromatographic techniques, etc. Further, a purification technique generally used in ordinary parallel syntheses (for example, combinatorial chemistry) using a scavenger resin such as benzoyl chloride polymer bound, poly-4-vinylpyridine, benzaldehyde polymer bound or trityl chloride polymer bound; an ion-exchange resin such as an AG1-X8 $OH^-$ resin (available from Bio-Rad Laboratories, Inc.); or

another resin. The intermediates can also be subjected to subsequent steps without purification.

In some of Compound (I), or the quaternary ammonium salts thereof, or the pharmaceutically acceptable salts thereof, there can be isomers such as regioisomers, geometrical isomers, optical isomers, etc. All possible isomers including these isomers, and mixtures of the isomers in any ratio are within the scope of the present invention.

In order to obtain a salt of Compound (I) or a quaternary ammonium salt thereof, when Compound (I) or the quaternary ammonium salt thereof is obtained in the form of a salt, the salt may be directly purified. Alternatively, when Compound (I) or the quaternary ammonium salt thereof is obtained in a free form, the corresponding salt can be obtained by dissolving or suspending Compound (I) in an appropriate solvent and adding an acid or a base thereto.

Compound (I), or the quaternary ammonium salts thereof, or the pharmaceutically acceptable salts thereof may exist in the form of adducts with water or any solvent. These adducts are also within the scope of the present invention.

Tables 1 to 6 show examples of Compound (I) and the quaternary ammonium salts thereof. The present invention is not limited to such examples. In the tables below, Et represents ethyl.

**Table 1**

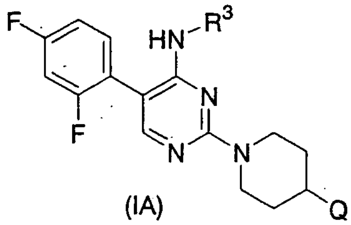

(IA)

| Compound Number | •—$R^3$ | •—Q |
|---|---|---|
| 1 | 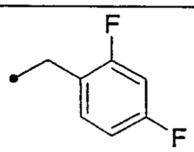 | 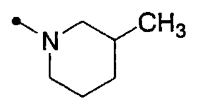 |
| 2 | 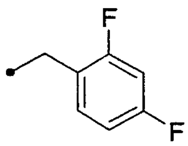 | 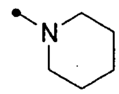 |
| 3 | 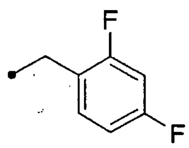 | 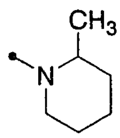 |
| 4 | 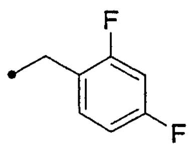 | 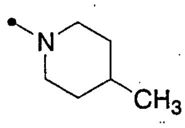 |
| 5 | 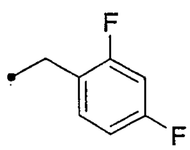 | 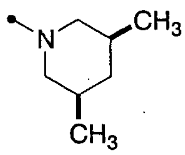 |
| 6 | 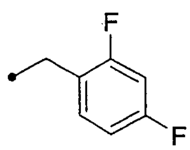 | 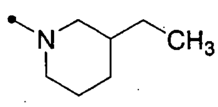 |
| 7 | 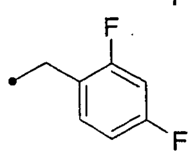 | 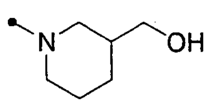 |
| 8 | 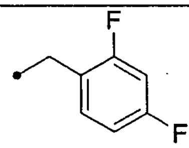 | 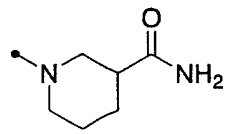 |
| 9 | 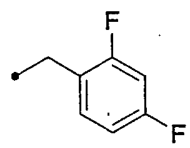 | 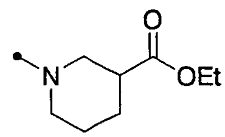 |
| 10 | 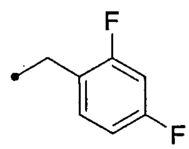 | 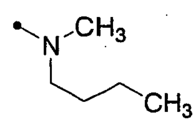 |
| 15 | 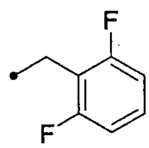 | 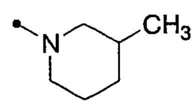 |
| 16 | 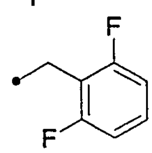 | 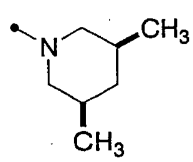 |

**Table 2**

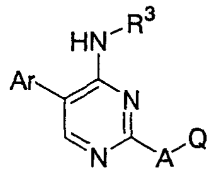

(IB)

| Compound Number | •—Ar | •—$R^3$ | •—A—Q |
|---|---|---|---|
| 11 | 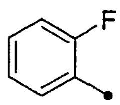 | 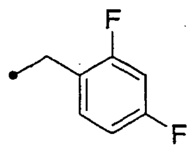 | 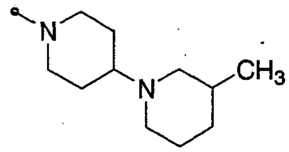 |
| 12 | 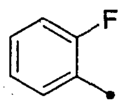 | 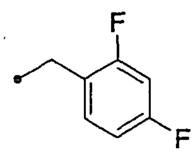 | 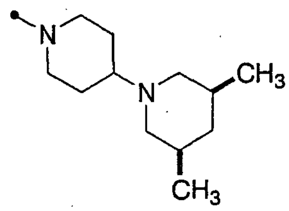 |
| 13 | 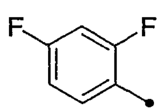 | 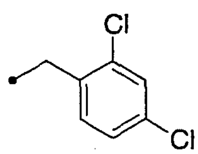 | 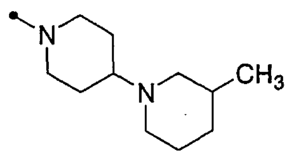 |
| 14 | 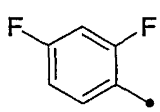 | 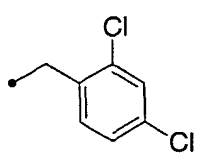 | 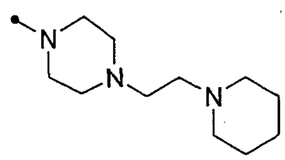 |
| 17 | 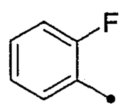 | 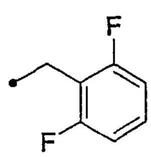 | 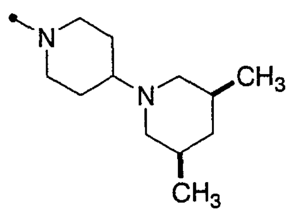 |

**Table 3**

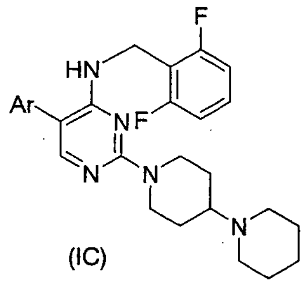

(IC)

| Compound Number | •—Ar | Compound Number | •—Ar |
|---|---|---|---|
| 18 | 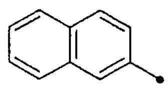 | 27 | 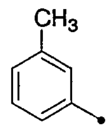 |
| 19 | 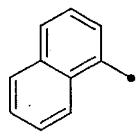 | 28 | 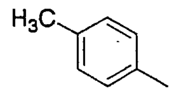 |
| 20 | 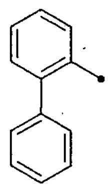 | 29 | 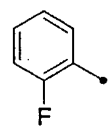 |
| 21 | 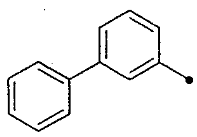 | 30 | 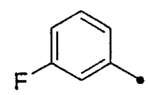 |
| 22 | 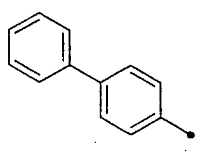 | 31 | 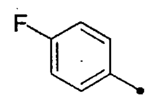 |
| 23 | 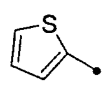 | 32 | 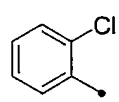 |
| 24 | 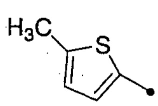 | 33 | 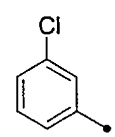 |
| 25 | 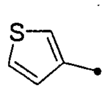 | 34 | 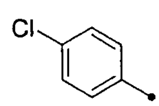 |
| 26 | 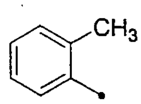 | 35 | 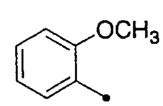 |

**Table 3 (Continued)**

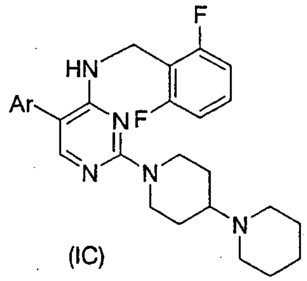

(IC)

| Compound Number | •—Ar | Compound Number | •—Ar |
|---|---|---|---|
| 36 | 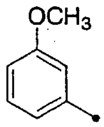 | 44 | 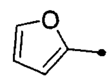 |
| 37 | 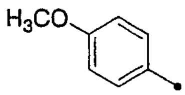 | 45 | 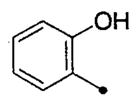 |
| 38 | 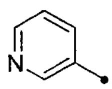 | 46 | 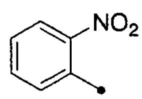 |
| 39 | 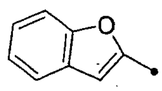 | 47 | 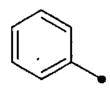 |
| 40 | 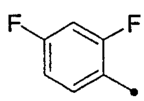 | 48 | 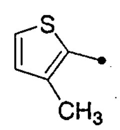 |
| 41 | 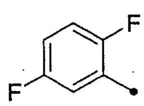 | 49 | 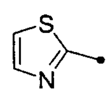 |
| 42 | 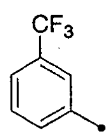 | 50 | 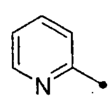 |
| 43 | 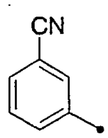 | | |

**Table 4**

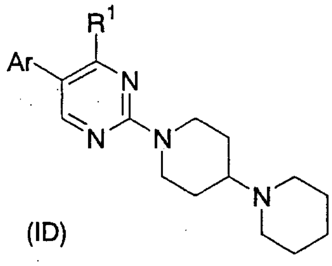

(ID)

| Compound Number | •—Ar | •—$R^1$ |
|---|---|---|
| 51 | 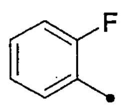 | 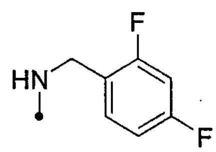 |
| 52 | 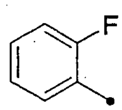 | 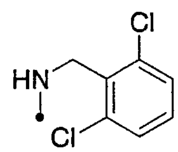 |
| 53 | 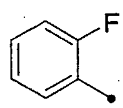 | 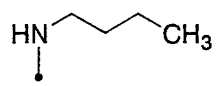 |
| 54 | 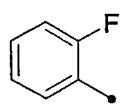 | 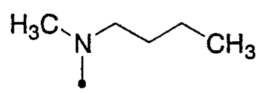 |
| 55 | 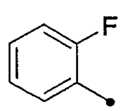 | 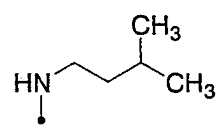 |
| 56 | 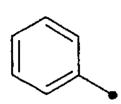 | 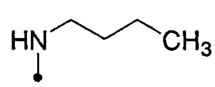 |
| 57 | 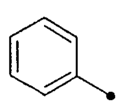 | 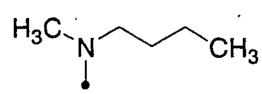 |

## Table 5

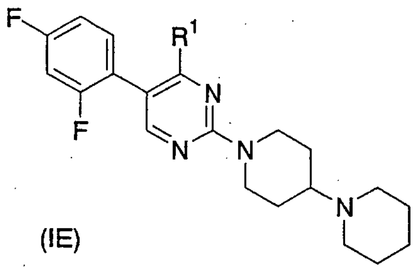

(IE)

| Compound Number | •—$R^1$ |
|---|---|
| 58 | 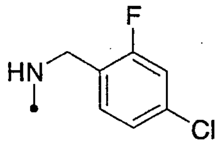 |
| 59 | 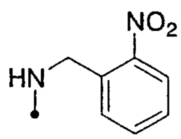 |
| 60 | 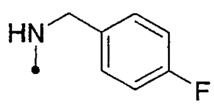 |
| 61 | 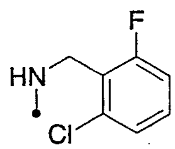 |
| 62 | 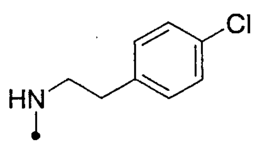 |
| 63 | 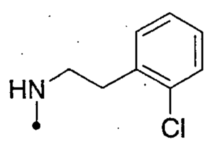 |
| 64 | 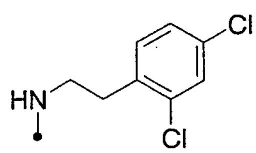 |
| 65 | 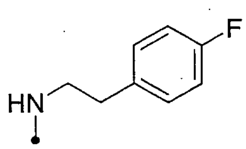 |
| 66 | 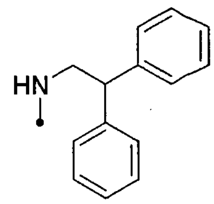 |
| 67 | 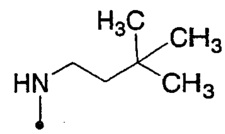 |
| 68 | 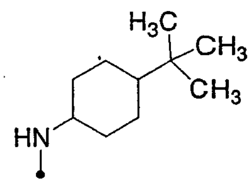 |
| 69 | 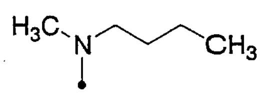 |
| 70 | 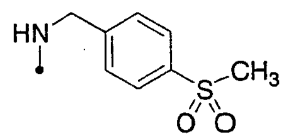 |
| 71 | 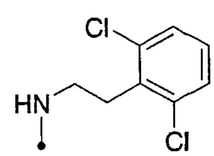 |

**Table 6**

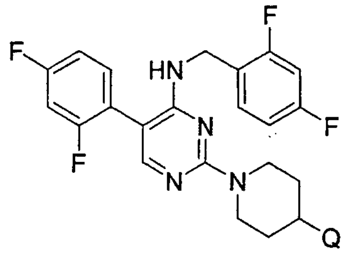

| Compound Number | •—$R^1$ |
|---|---|
| 72 | 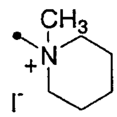 |
| 73 | 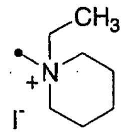 |
| 74 | 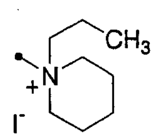 |
| 75 | 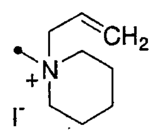 |
| 76 | 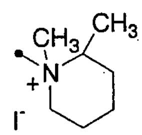 |
| 77 | 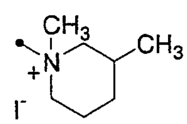 |

Typical pharmacological activities of Compound (I) will now be described in detail with reference to test examples. Test example 1: Inhibitory activity on binding of [$^{125}$I]-TARC to Hut78 cells

The RPMI-1640 medium (Sigma-Aldrich Japan) containing 20 mmol/L HEPES [4-(2-hydroxyethyl)-1-piperazinylethanesulfonic acid; HEPES, Nacalai Tesque, Inc.] and 0.1 w/v% bovine serum albumin (SEIKAGAKU CORPORATION) was adjusted to pH 7.0 with sodium hydrogencarbonate (Wako Pure Chemical Industries, Ltd.) to yield a binding/wash buffer. In a 96-well round-bottomed plate (Corning Costar Corporation) were placed 60 μL of a suspension of Hut78 cells (ATCC No. TIB-161) (3 x $10^5$ cells) in the binding/wash buffer, 20 μL of a solution of a test compound and 20 μL of a solution of 810 Bq of [$^{125}$I]-TARC (Amersham Biosciences K.K.) diluted with the binding/wash buffer up to the total aliquot of 100 μL, followed by reaction at room temperature for two hours. The solution of the test compound had been prepared by dissolving the test compound in dimethyl sulfoxide (Nacalai Tesque, Inc.) to a concentration of 10 mmol/L, and diluting the solution with the binding/wash buffer to a series of concentrations. The nonspecific binding was determined by a binding assay in the presence of a sufficient amount of unlabeled TARC. The total binding was defined as a binding in the case where dimethyl sulfoxide (Nacalai Tesque, Inc.) was added, instead of the test compound, in the same concentration (0.1 v/v%). After binding [$^{125}$I]-TARC to Hut78 cells, 50 μL of a polyethyleneimine solution (Nacalai Tesque, Inc.) diluted to 0.3 w/v% with the binding/wash buffer was added each of wells of a glass filter (Unifilter GF/B96, Packard BioScience Company), was rapidly filtrated using Filtermate 196 (Packard BioScience

Company) and washed with the binding/wash buffer at 4°C to thereby separate the radioactive ligand which is not bound to the cells. Microscinti 20 (Packard BioScience Company) was placed in each well at 50 μL/well, and the radioactivity on the glass filter was determined using Topcount NXT™ (Packard BioScience Company). The binding inhibitory rates of the test compounds at 1 μmol/L are shown in Table 7. The binding inhibitory rates (%) of the test compounds were determined by calculation according to the following equation:

$$\text{Binding inhibitory rate (\%)} = \frac{\text{(Total binding) - (Binding upon addition) of test compound}}{\text{(Total binding) - (Nonspecific binding)}} \times 100$$

| | |
|---|---|
| Total binding: | [$^{125}$I]-TARC binding radioactivity without addition of the test compound |
| Binding upon addition of test compound: | [$^{125}$I]-TARC binding radioactivity at respective concentration upon addition of test compound |
| Nonspecific binding: | [$^{125}$I]-TARC binding radioactivity upon addition of unlabeled TARC |

Table 7

| Compound Number | The Binding Inhibitory Rates at 1 μmol/L (%) |
|---|---|
| 1 | 91 |
| 3 | 93 |
| 8 | 92 |
| 15 | 90 |
| 40 | 89 |
| 58 | 81 |
| 61 | 82 |
| 72 | 96 |
| 73 | 99 |
| 75 | 97 |

These results show that Compound (I) of the present invention or quaternary ammonium salt thereof have excellent inhibitory activity on the binding of TARC to Hut 78 cells.

Chemotaxing cells by TARC or MDC are known to have high IL-4 productivity and low IFN-γ productivity [International Immunology, vol. 11, p. 81 (1999)]. More specifically, Th2 T cells are considered to play an important role on allergic reactions, and CCR4 as well as TARC and MDC as ligands thereof are considered to be significantly involved in allergic pathoses [Molecular Immunology, vol. 38, p. 881 (2002); and Allergy, vol. 57, p. 180 (2002)].

It has been reported, for example, that increase of CCR4-positive T cells in the peripheral blood [American Journal of Respiratory and Critical Care Medicine, vol. 164, p. 754 (2001)], increase of TARC in the peripheral blood and sputum [Allergy, vol. 57, p. 173 (2002)], and invasion of CCR4-positive T cells in the lung tissues by antigenic stimulation [Journal of Clinical Investigation, vol. 107, p. 1357 (2001)] are shown in a patient of asthma. Asthmatic pathosis is strongly prevented in mice administered with an antibody against TARC [Journal of Immunology, vol. 166, p. 2055 (2001)], and an anti-MDC antibody shows inhibitory activities on asthmatic pathosis in a murine asthma model [Journal of Immunology, vol. 163, p. 403 (1999)].

Eosinophilic pneumonia patients show increase in TARC in the peripheral blood and the bronchoalveolar lavage [American Journal of Respiratory and Critical Care Medicine, vol. 165, p. 1125 (2002)].

Rhinitis patients are known to show increase in TARC level in the peripheral blood [Allergy, vol. 57, p. 180 (2002)], and a CCR4 ligand TARC is known to be produced from the human nasal mucosa [Clinical and Experimental Allergy, vol. 31, p. 1923 (2001)].

It has been reported that the production of TARC is increased by stimulating the corneal fibroblasts with a Th2 cytokine such as IL-4 or TNF-α, indicating that CCR4-positive cells may participate in allergic conjunctivitis [Biochemical and Biophysical Research Communications, vol. 279, p. 1 (2000)]. It has been reported in psoriasis that CCR4-positive cells increasingly invade around the cutaneous blood vessel in lesions [Laboratory Investigation, vol. 81, p. 335 (2001)]. It has also been reported that CCR4-positive cells invade the synovial membrane tissue in rheumatoid arthritis [Arthritis and Rheumatism, vol. 44, p. 2750 (2001)], and that CCR4-positive cells increase in the peripheral blood of systemic lupus erythematosus patients in stages with high disease activity [Journal of Leukocyte Biology, vol. 70, p. 749 (2001)].

CCR4 is expressed in the nerve cells, particularly in substance P neurons in the dorsal root ganglion, and the

stimulation of the nerve cells by MDC increases an intracellular calcium level to thereby release substance P serving as a pain producing substance [Journal of Neuroscience, vol. 21, p. 5027 (2001)]. CCR4 knockout mice become resistant against sepsis [Journal of Experimental Medicine, vol. 191, p. 1755 (2000)]. Further, cells in which CCR4 is expressed were known in leukemia [Blood, vol. 96, p. 685 (2000)], and CCR4 is significantly expressed in leukemia cells particuraly in adult T cell leukemia (ATL) [Blood, vol. 99, p. 1505 (2002)].

Atopic dermatitis patients have been reported to show an increasing TARC level in the peripheral blood according with an disease severity [Journal of Allergy Clinical Immunology, vol. 107, p. 535 (2001)] and an increasing MDC level in the peripheral blood with an increasing severity [Clinical and Experimental Immunology, vol. 127, p. 270 (2002)]. Further, atopic dermatitis patients have been reported to show an increased proportion of CCR4-positive cells in CD4-positive T cells in the peripheral blood [Journal of Investigative Dermatology, vol. 117, p. 188 (2001)].

It has been reported that an anti-mouse MDC antibody inhibits the onset of diabetes mellitus in a murine insulin-dependent diabetes mellitus model (NOD mice) [Journal of Clinical Investigation, vol. 110, p. 1675 (2002)].

In patients infected by *Candida albicans* in the intraoral skin, it has been reported that CCR4-positive T cells and MDC producing dendritic cells increase in the epidermis and dermis in regions of inflammation caused by infection [American Journal of Pathology, vol. 158, p. 1263 (2000)].

TARC has been reported to participate in pathoses in mycosis fungoides [Journal of American Academy of Dermatology, vol. 48, p. 23 (2003)].

Compound (I) of the present application, or the quaternary ammonium salts thereof, or the pharmaceutically acceptable salts thereof regulate or control the functions of TARC and/or MDC, namely, inhibit or antagonize the binding of TARC and/or MDC to T cells, and more specifically, for example, inhibit or antagonize the binding of TARC and/or MDC to CCR4 (i.e., have CCR4 antagonistic actions). Based on the above-mentioned findings, Compound (I) of the present application, or the quaternary ammonium salts thereof, or the pharmaceutically acceptable salts thereof are considered to be promising agents for treating, for example, allergic diseases and to be particularly efficacious for treating, for example, asthma, rhinitis, allergic conjunctivitis and atopic dermatitis.

Compound (I) of the present application, or the quaternary ammonium salts thereof, or the pharmaceutically acceptable salts thereof are considered to be promising agents for treating malignant lymphoma, leukemia and the like. In particular, Compound (I) of the present application, or the quaternary ammonium salts thereof, or the pharmaceutically acceptable salts thereof are considered to be effective for treating adult T-cell leukemia (ATL), cutaneous T-cell lymphoma, and the like.

Furthermore, Compound (I) of the present application, or the quaternary ammonium salts thereof, or the pharmaceutically acceptable salts thereof are considered to be effective for treating eosinophilic pneumonia, eosinophilic sinusitis, rhinitis with eosinophilia, endometriosis, Churg-Strauss syndrome, psoriasis, articular rheumatism, systemic lupus erythematosus, sepsis, interstitial cystitis, insulin-dependent diabetes mellitus (IDDM), oral candidiasis, mycosis fungoides, contact dermatitis, urticaria, and the like.

Compound (I) of the present application, or the quaternary ammonium salts thereof, or the pharmaceutically acceptable salts thereof are also considered to be effective for alleviating pain and/or neuralgia by inhibiting the liberation of pain-producing substances, treating cutaneous itching and the like.

Test Example 2: Inhibitory activities on antigen-induced cellular infiltration and cytokine production

BALB/c mice were sensitized by intraperitoneally administrating 50 µg of ovalbumin (Sigma-Aldrich Japan) and 1 mg of aluminum hydroxide (Wako Pure Chemical Industries, Ltd.) and further sensitized in the same manner on the 7th day from the first sensitization (0 day). From on the 23rd day, the mice were allowed to inhale a 1% ovalbumin physiological saline solution [prepared by dissolving ovalbumin in physiological saline (Otsuka Pharmaceutical Co., Ltd.) to a concentration 1%] using Supersonic Nebulizer NE-U 12 (OMRON Corporation) for 30 minutes once per 2 days (antigen nebulization). DMSO (Kanto Kagaku), Tween 80 (Wako Pure Chemical Industries), and 10 mmol/L acetic acid buffer containing 0.9% sodium chloride were mixed at a ratio of 5 to 5 to 90, whereby a liquid (an administration solvent) was prepared. Each test compound was dissolved in the administration solvent, whereby a solution for subcutaneous administration was prepared. The mice were grouped into test compound-administered groups and a control group. The test compound was subcutaneously administered to the mice of each of the test compound-administered groups at a dose of 30 mg/kg and the administration solvent was subcutaneously administered to the mice of the control group 30 minutes prior to the fourth antigen nebulization and after 4 hours from the fourth antigen nebulization. 8 hours after the fourth antigen nebulization, the bronchoalveolar lavage of each mouse was performed to obtain a bronchoalveolar lavage fluid. The number of CD4-positive T cells and eosinophils in the bronchoalveolar lavage fluid were counted. 10 mice were subjected to the each group in the test.

For the control group, the number of CD4-positive T cells in a bronchoalveolar lavage fluid obtained from each mouse was $(1.9 \pm 0.2) \times 10^4$ (mean ± standard error). For a Compound 8-administered group, the number was $(0.6 \pm 0.1) \times 10^4$ (mean ± standard error). The number of CD4-positive T cells of the Compound 8-administered group

is 68% less than that of the control group ($P < 0.0001$, Aspin-Welch test).

For the control group, the number of eosinophils in a bronchoalveolar lavage fluid obtained from each mouse was $(1.5 \pm 0.1) \times 10^5$ (mean ± standard error). For the Compound 8-administered group, the number was $(0.6 \pm 0.1) \times 10^5$ (mean ± standard error). The number of eosinophils of the Compound 8-administered group is 57% less than that of the control group ($P < 0.0001$, Student's test).

The excellent pharmacological activities of the compounds of the present invention can also be shown, in addition to the above test examples, by evaluation models generally used for determining anti-inflammatory activities [for example, a guinea pig asthma model disclosed in *Journal of Pharmacology and Experimental Therapeutics,* vol. 278, p. 847 (1996); a mouse respiratory hypersensitivity model disclosed in *Journal of Immunology,* vol. 163, p. 403 (1999); a delayed type hypersensitivity model disclosed in *Journal of Immunology,* vol. 167, p. 3980 (2001); and a collagen arthritis model disclosed in *Journal of Immunology,* vol. 167, p. 1004 (2001)].

Compound (I), or quaternary ammonium salts thereof, or pharmacologically acceptable salts thereof can be administered alone as intact, however is preferably administered as pharmaceutical preparations in general. Such pharmaceutical preparations can be used for animals or humans.

The pharmaceutical preparations according to the present invention may comprise, as an active ingredient, Compound (I), or a quaternary ammonium salt thereof, or a pharmaceutically acceptable salt thereof alone or optionally as a mixture with any other active ingredient for treatment. The pharmaceutical preparations are produced by mixing the active ingredient with one or more pharmaceutically acceptable carries and formulating them according to any procedure known in the technical field of pharmaceutics.

The administration route is preferably most efficacious one in the treatment and includes oral administration or parenteral administration such as intravenous administration.

The dosage form includes, for example, tablets and injections.

Examples of the carriers for the pharmaceutical preparations include lactose, mannitol, glucose, hydroxypropylcellulose, starch, magnesium stearate, sorbitan fatty acid esters, glyceric acid esters, polyvinyl alcohol, distilled water for injection, physiological saline, propylene glycol, polyethylene glycol, ethanol and the like. The pharmaceutical preparations according to the present invention may further comprise any of various excipients, lubricants, binders, disintegrators, isotonizing agents, emulsifiers and the like.

In order to use Compound (I), or the quaternary ammonium salt, or the pharmaceutically acceptable salt thereof for the above purposes, it is usually administered to patients systemically or locally, orally or parenterally. The dose and frequency of administration vary depending on the dosage form, the age and weight of patients, and properties or the severity of symptoms to be treated. Compound (I), or the quaternary ammonium salt, or the pharmaceutically acceptable salt thereof is preferably administered to patients at a dose of 0.1 to 100 mg/kg and preferably 1 to 50 mg/kg per day per one adult in three to four installments. However, the dose and frequency of administration vary depending of the above conditions.

## Best Mode for Carrying Out the Invention

The present invention will be described in detail with reference to Examples and Reference Examples below, which are never intended to limit the scope of the present invention. The numbers of compounds used in Examples and Reference Examples correspond to the numbers of compounds listed in Tables 1 to 6.

Intermediates of the compounds listed in Tables 1 to 6 were synthesized according to Reference Examples descrobed below.

### Reference Example 1

2-chloro-4-(2,4-difluorobenzylamino)-5-(2,4-difluorophenyl)pyrimidine (Compound A1)

Step 1

To 2,4-dichloro-5-iodopyrimidine (4.00 g, 14.6 mmol), 2,4-difluorobenzylamine (2.26 mL, 19.0 mmol) and ethanol (44.2 mL) were added and the mixture was heated and stirred for 6 hours under reflux. A saturated aqueous sodium hydrogencarbonate solution was added to the reaction mixture, followed by extraction with ethyl acetate. The organic layer was washed with saturated brine and then dried over anhydrous sodium sulfate, followed by evaporation of the solvent. The residue was triturated with diisopropyl ether and a small amount of isopropyl alcohol, and then the solid was collected by filtration to obtain 2-chloro-4-(2,4-difluorobenzyl)-5-iodopyrimidine (Compound A1a) (3.44 g, 62%).

Step 2

Compound A1a (3.04 g, 7.96 mmol) obtained in the above step was dissolved in degassed dimethoxyethane (26.5 ml) under argon atmosphere, and the solution was added with dihydroxy(2,4-difluorophenyl)borane (1.63 g, 10.3 mmol), cesium carbonate (6.42 g, 20.0 mmol) and [1,1'-bis(diphenylphosphino)ferrocene]palladium (II) dichloride-dichloromethane complex (0.211 g, 0.258 mmol). The mixture was heated and stirred for 12 hours under reflux. The reaction mixture was cooled then added with water, followed by extraction with ethyl acetate. The organic layer was washed with saturated brine and then dried over anhydrous sodium sulfate, followed by evaporation of the solvent. Impurities on the start line of a thin-layer chromatography (TLC) plate were removed by purification of the residue using column chromatography (ethyl acetate : hexane = 10:90 to 80:20), and the elute was concentrated. The residue was triturated with isopropyl ether and the solid was collected by filtration to obtain Compound A1 (1.86 g, 64%).
$^1$H NMR ($CDCl_3$) $\delta$ (ppm): 4.66 (d, J = 5.9 Hz, 2H), 5.10-5.40 (m, 1H), 6.70-7.13 (m, 4H), 7.20-7.50 (m, 2H), 7.88 (s, 1H).

Reference Example 2

2-chloro-4-(2,4-difluorobenzylamino)-5-(2-fluorophenyl)pyrimidine (Compound A2)

Compound A2 (96 mg, 65%) was obtained using Compound A1a obtained in Step 1 of Reference Example 1 and dihydroxy(2,4-difluorophenyl)borane in a similar manner to Step 2 of Reference Example 1.
$^1$H NMR ($CDCl_3$) $\delta$ (ppm): 4.67 (d, J = 9.3 Hz, 2H), 5.10-5.44 (m, 1H), 6.70-6.95 (m, 2H), 7.10-7.57 (m, 5H), 7.91 (s, 1H).

Reference Example 3

2-chloro-4-(2,4-dichlorobenzylamino)-5-(2,4-difluorophenyl)pyrimidine (Compound A3)

2-chloro-4-(2,4-dichlorobenzyl)-5-iodopyrimidine (Compound A3a) was obtained using 2,4-dichloro-5-iodopyrimidine and 2,4-dichlorobenzylamine and Compound A3 (0.286 g, 56% through 2 steps) was then obtained from Compound A3a and dihydroxy(2,4-difluorophenyl)borane in a similar manner to Reference Example 1.
$^1$H NMR ($CDCl_3$) $\delta$ (ppm): 4.70 (d, J = 5.1 Hz, 2H), 5.20-5.40 (m, 1H), 6.80-7.13 (m, 2H), 7.15-7.65 (m, 4H), 7.91 (s, 1H). Reference Example 4

5-bromo-4-(2,6-difluorobenzylamino)-2-[4-(3-methylpiperidino)piperidino]pyrimidine (Compound B1)

Step 1

5-bromo-2-chloro-4-(2,6-difluorobenzylamino)pyrimidine (Compound B1a) (0.54 g, 81%) was obtained using 5-bromo-2,4-dichloropyrimidine and 2,6-difluorobenzylamine in a similar manner to Step 1 of Reference Example 1.

Step 2

Compound B1a (120 mg, 0.358 mmol) obtained in the above step was dissolved in DMF (0.2 mL), and the solution was added with 4-(3-methylpiperidino)piperidine (81 mg, 0.44 mmol) obtained according to the procedure described in the literature [*J. Org. Chem.*, vol. 55, p. 2552 (1990)] and Triethylamine (50 μL, 0.36 mmol). The mixture was stirred at 120°C for 6 hours. A saturated aqueous sodium hydrogencarbonate solution was added to the reaction mixture, followed by extraction with ethyl acetate. The organic layer was washed with saturated brine and then dried over anhydrous sodium sulfate, followed by evaporation of the solvent. The residue was purified by column chromatography (methanol : chloroform : triethylamine = 3:97:1 to 20:80:1) to obtain Compound B1 (0.107 g, 62%).
$^1$H NMR ($CDCl_3$) $\delta$ (ppm): 0.70-0.95 (m, 4H), 1.35-1.95 (m, 8H), 2.09 (td, J = 11.7, 3.4 Hz, 1H), 2.44-2.94 (m, 6H), 4.60-4.90 (m, 4H), 5.54 (t, J = 6.0 Hz, 1H), 6.78-6.95 (m, 2H), 7.13-7.30 (m, 1H), 7.85 (s, 1H).

Reference Example 5

5-bromo-4-(2,6-difluorobenzylamino)-2-(4-piperidinopiperidino)pyrimidine (Compound B2)

Compound B2 (3.3 g, 85%) was obtained using Compound B1a obtained in Step 1 of Reference Example 4 and 4-piperidinopiperidine in a similar manner to Step 2 of Reference Example 4.
$^1$H NMR ($CDCl_3$) $\delta$ (ppm): 1.35-1.75 (m, 8H), 1.77-1.92 (m, 2H), 2.38-2.61 (m, 5H), 2.77 (dt, J = 1.8, 13 Hz, 2H), 4.74 (d, J = 6.1 Hz, 2H), 4.77-4.83 (m, 2H), 5.52 (t, J = 6.1 Hz, 1H), 6.80-6.95 (m, 2H), 7.15-7.32 (m, 1H), 7.88 (s, 1H).

APCIMS m/z: $[M + H]^+$ 466

Reference Example 6

4-(2,6-difluorobenzylamino)-2-[4-(cis-3,5-dimethylpiperidino)piperidino]-5-iodopyrimidine (Compound B3)

2-chloro-4-(2,6-difluorobenzylamino)-5-iodopyrimidine (Compound B3a) was obtained using 2,4-dichloro-5-iodopyrimidine and 2,6-difluorobenzylamine in a similar manner to Step 1 of Reference Example 1, and Compound B3 (0.150 g, 53% through 2 steps) was then obtained using Compound B3a and 4-(cis-3,5-dimethylpiperidino)piperidine obtained according to a procedure disclosed in a literature *[J. Org. Chem.*, vol. 55, p. 2552 (1990)].
$^1$H NMR ($CDCl_3$) δ (ppm): 0.40-0.65 (m, 1H), 0.80-0.98 (m, 7H), 1.36-2.00 (m, 7H), 2.47-2.94 (m, 6H), 4.62 (d, J = 8.4 Hz, 2H), 4.80-4.83 (m, 2H), 4.95 (t, J = 6.8 Hz, 1H), 6.80-7.00 (m, 2H), 7.13-7.35 (m, 1H), 7.84 (s, 1H).

Reference Example 7

2-chloro-4-(2,6-difluorobenzylamino)-5-(2-nitrophenyl)pyrimidine (Compound E1)

Step 1

Dihydroxy(2,4-di-tert-butoxypyrimidine-5-yl)borane (0.357 g, 1.33 mmol) and 1-iodo-2-nitrobenzene (0.222 g, 0.893 mmol) were dissolved in degassed toluene (4.4 mL) under argon atmosphere, and the solution was added with Tetrakis(triphenylphosphine)palladium (0) (0.105 g, 0.0893 mmol) and a 1 mol/L aqueous sodium hydrogencarbonate solution (3.3 mL), followed by stirring at 100°C for 1 hour. Tetrakis(triphenylphosphine)palladium (0) (0.101 g, 0.0877 mmol) was further added to the reaction mixture, followed by stirring at 100°C for 63 hours. Water was added to the reaction mixture, followed by extraction with ethyl acetate. The organic layer was washed with saturated brine and then dried over anhydrous sodium sulfate, followed by evaporation of the solvent. The residue was purified by column chromatography (ethyl acetate : hexane = 10:90) to obtain 2,4-di-tert-butoxy-5-(2-nitrophenyl)pyrimidine (Compound E1a).

To Compound E1a obtained in the above step, 6 mol/L hydrochloric acid (1.8 mL) and methanol (1.8 mL) were added, followed by stirring at room temperature for 40 minutes. The reaction mixture was concentrated, and deposited crystals were collected by filtration. The crystals were washed with ethanol, water and chloroform in that order. The filtrate obtained by the filtration was adjusted to pH 6 to 5 with an aqueous sodium hydroxide solution and then subjected to extraction with chloroform. The organic layer was washed with saturated brine and then dried over anhydrous sodium sulfate, followed by evaporation of the solvent. The residue was reslurried with ethanol and crystals were collected by filtration. The crystals were mixed with the crystals previously obtained. The crystal mixture was dried under reduced pressure to obtain 5-(2-nitrophenyl)-1H,3H-pyrimidine-2,4-dione (Compound E1b) (0.148 g, 71% through 2 steps).

Step 2

Diisopropylethylamine (6.36 mL) and phosphorus oxychloride (3.2 mL) were added to Compound E1b (0.370 g, 1.59 mmol) obtained in the above step, followed by stirring at 110°C for 27 hours. Water was added to the reaction mixture, followed by extraction with ethyl acetate. The organic layer was washed with a saturated aqueous sodium hydrogencarbonate solution and saturated brine, and then dried over anhydrous sodium sulfate, followed by evaporation of the solvent. The residue was purified by column chromatography (chloroform) to obtain 2,4-dichloro-5-(2-nitrophenyl)pyrimidine (Compound E1c) (95 mg, 22%).

Step 3

Compound E1c (0.105 g, 0.390 mmol) obtained in the above step was dissolved in ethanol (2.6 mL), and the solution was added with triethylamine (0.435 mL, 3.12 mmol) and 2,6-difluorobenzylamine (56.0 μL, 0.468 mmol), followed by stirring at 80°C for 9 hours. The reaction mixture was concentrated and then added with water, followed by extraction with ethyl acetate. The organic layer was washed with water and then dried over anhydrous sodium sulfate, followed by evaporation of the solvent. The residue was purified by column chromatography (ethyl acetate : hexane : triethylamine = 5:95:10 to 40:60:10) to obtain Compound E1 (26 mg, 18%).
$^1$H NMR ($CDCl_3$) δ (ppm): 4.55-4.92 (m, 3H), 6.73-7.05 (m, 2H), 7.07-7.45 (m, 2H), 7.46-7.80 (m, 2H), 7.85-8.27 (m, 2H).

Reference Example 8

2-chloro-4-(2,6-difluorobenzylamino)-5-phenylpyrimidine (Compound E2)

2,4-dichloro-5-phenylpyrimidine (Compound E2c) was obtained using dihydroxy(2,4-di-tert-butoxypyrimidine-5-yl)borane and bromobenzene in a similar manner to Step 1 and Step 2 of Reference Example 7. Compound E2 (66 mg, 44% through 4 steps) was then obtained using Compound E2c and 2,6-difluorobenzylamine in a similar manner to Step 3 of Reference Example 7.
$^1$H NMR ($CDCl_3$) δ (ppm): 4.75 (d, J = 5.6 Hz, 2H), 5.49 (brs, 1H), 6.82-6.98 (m, 2H), 7.15-7.56 (m, 6H), 7.90 (s, 1H).
APCIMS m/z: $[M + H]^+$ 332

Reference Example 9

2-chloro-4-(2,6-difluorobenzylamino)-5-(3-methyl-2-thienyl)pyrimidine (Compound E3)

2,4-dichloro-5-(3-methyl-2-thienyl)pyrimidine (Compound E3c) was obtained using dihydroxy(2,4-di-tert-butoxypyrimidine-5-yl)borane and 2-bromo-3-methylthiophene in a similar manner to Step 1 and Step 2 of Reference Example 7. Compound E3 (42 mg, 8% through 4 steps) was then obtained using Compound E3c and 2,6-difluorobenzylamine in a similar manner to Step 3 of Reference Example 7.
$^1$H NMR ($CDCl_3$) δ (ppm): 2.05 (s, 3H), 4.75 (d, J = 5.7 Hz, 2H), 5.40 (brs, 1H), 6.89 (t, J = 7.8 Hz, 2H), 6.95 (d, J = 4.9 Hz, 1H), 7.25 (t, J = 8.1 Hz, 1H), 7.34 (d, J = 5.4 Hz, 1H), 7.91 (s, 1H).

Reference Example 10

2-chloro-4-(2,6-difluorobenzylamino)-5-(2-thiazolyl)pyrimidine (Compound E4)

2,4-dichloro-5-(2-thiazolyl)pyrimidine (Compound E4c) was obtained using dihydroxy(2,4-di-tert-butoxypyrimidine-5-yl)borane and 2-bromothiazole in a similar manner to Step 1 and Step 2 of Reference Example 7. Compound E4 (42 mg, 22% through 4 steps) was then obtained using Compound E4c and 2,6-difluorobenzylamine in a similar manner to Step 3 of Reference Example 7.
$^1$H NMR ($CDCl_3$) δ (ppm): 4.91 (d, J = 5.6 Hz, 2H), 6.85-7.00 (m, 2H), 7.18-7.40 (m, 2H), 7.80 (d, J = 3.5 Hz, 1H), 8.50 (s, 1H), 9.9 (brs, 1H).
APCIMS m/z: $[M + H]^+$ 339

Reference Example 11

2-chloro-4-(2,6-difluorobenzylamino)-5-(2-pyridyl)pyrimidine (Compound E5)

2,4-dichloro-5-(2-pyridyl)pyrimidine (Compound E5c) was obtained using dihydroxy(2,4-di-tert-butoxypyrimidine-5-yl)borane and 2-bromopyrimidine in a similar manner to Step 1 and Step 2 of Reference Example 7. Compound E5 (11 mg, 13% through 4 steps) was then obtained using Compound E5c and 2,6-difluorobenzylamine in a similar manner to Step 3 of Reference Example 7.
$^1$H NMR ($CDCl_3$) δ (ppm): 4.90 (d, J = 5.6 Hz, 2H), 6.85-6.98 (m, 2H), 7.18-7.35 (m, 2H), 7.72-7.83 (m, 2H), 8.52 (s, 1H), 8.55 (d, J = 4.9 Hz, 1H), 10.5 (brs, 1H).
APCIMS m/z: $[M + H]^+$ 333

Reference Example 12

4-chloro-5-(2-fluorophenyl)-2-methylthiopyrimidine (Compound F1a)

The mixture of 4-chloro-5-iodo-2-methylthiopyrimidine (1.0 g, 3.5 mmol), dihydroxy(2-fluorophenyl)borane (0.59 g, 4.2 mmol), dichlorobis(tri-o-tolylphosphine)palladium (II) (0.14 g, 0.18 mmol), a 1 mol/L aqueous sodium hydrogencarbonate solution (8.2 mL) and dimethoxyethane (20 mL) was heated and stirred under reflux for 4 hours. After the mixture was cooled, water was added to the mixture, followed by extraction with ethyl acetate. The organic layer was washed with saturated brine and then dried over anhydrous sodium sulfate, followed by evaporation of the solvent. The residue was purified by column chromatography (hexane : diethyl ether = 30:1) to obtain Compound F1a (0.34 g, 38%).
$^1$H NMR ($CDCl_3$) δ (ppm): 2.62 (s, 3H), 7.10-7.55 (m, 4H), 8.41 (s, 1H).

APCIMS m/z: $[M + H]^+$ 255

Reference Example 13

4-chloro-2-methylthio-5-phenylpyrimidine (Compound F2a)

Compound F2a (0.31 g, 54%) was obtained using 4-chloro-5-iodo-2-methylthiopyrimidine (0.68 g, 2.4 mmol) and dihydroxy(phenyl)borane (0.29 g, 2.4 mmol) in a similar manner to Reference Example 12.
$^1$H NMR ($CDCl_3$) $\delta$ (ppm): 2.62 (s, 3H), 7.38-7.56 (m, 5H), 8.42 (s, 1H).
APCIMS m/z: $[M + H]^+$ 237

Reference Example 14

4-chloro-5-(2,4-difluorophenyl)-2-methylthiopyrimidine (Compound F3a)

Compound F3a (0.120 g, 44%) was obtained using 4-chloro-5-iodo-2-methylthiopyrimidine (0.300 g, 1.00 mmol) and dihydroxy(2,4-difluorophenyl)borane (0.210 g, 1.30 mmol) in a similar manner to Reference Example 12.
$^1$H NMR ($CDCl_3$) $\delta$ (ppm): 2.62 (s, 3H), 6.89-7.06 (m, 2H), 7.23-7.39 (m, 1H), 8.39 (s, 1H).
APCIMS m/z: $[M + H]^+$ 273

Example 1

4-(2,4-difluorobenzylamino)-5-(2,4-difluorophenyl)-2-[4-(3-methylpiperidino)piperidino]pyrimidine (Compound 1)

Compound A1 (77 mg, 0.21 mmol) obtained in Reference Example 1 was dissolved in DMF (0.42 mL), and the solution was added with 4-(3-methylpiperidino)piperidine dihydrochloride (0.123 g, 0.481 mmol) obtained according to a procedure disclosed in a literature *[J. Org. Chem.,* vol. 55, p. 2552 (1990)] and triethylamine (0.70 mL, 5.0 mmol), followed by stirring at 100°C for 9 hours. A saturated aqueous sodium hydrogencarbonate solution was added to the reaction mixture, followed by extraction with ethyl acetate. The organic layer was washed with saturated brine and then dried over anhydrous sodium sulfate, followed by evaporation of the solvent. The residue was purified by column chromatography (methanol : chloroform = 3:97 to 5:95) to obtain Compound 1 (57 mg, 53%).
$^1$H NMR ($CDCl_3$) $\delta$ (ppm): 0.88 (d, J = 6.1 Hz, 3H), 1.38-1.86 (m, 10H), 2.03-2.11 (m, 1H), 2.45-2.56 (m, 1H), 2.74-2.87 (m, 4H), 4.61 (d, J = 5.9 Hz, 2H), 4.76-4.86 (m, 3H), 6.73-6.96 (m, 4H), 7.18-7.35 (m, 2H), 7.74 (s, 1H).
APCIMS m/z: $[M+ H]^+$ 514

Example 2

4-(2,4-difluorobenzylamino)-5-(2,4-difluorophenyl)-2-(4-piperidinopiperidino)pyrimidine (Compound 2)

Compound 2 (0.246 g, 89%) was obtained using Compound A1 (0.203 g, 0.552 mmol) obtained in Reference Example 1 and 4-piperidinopiperidine in a similar manner to Example 1. $^1$H-NMR ($CDCl_3$) $\delta$ (ppm): 1.43-1.63 (m, 8H), 1.86-1.90 (m, 2H), 2.54-2.58 (m, 5H), 2.72-2.81 (t, J = 11.9 Hz, 2H), 4.62 (d, J = 5.9 Hz, 2H), 4.78 (d, J = 13.2 Hz, 2H), 5.12 (t, J = 5.9 Hz, 1H), 6.72-6.95 (m, 4H), 7.18-7.35 (m, 2H), 7.69 (s, 1H). APCIMS m/z: $[M + H]^+$ 500

Example 3

4-(2,4-difluorobenzylamino)-5-(2,4-difluorophenyl)-2-[4-(2-methylpiperidino)piperidino]pyrimidine (Compound 3)

Compound 3 (11 mg, 39%) was obtained using Compound A1 (21 mg, 0.057 mmol) obtained in Reference Example 1 and 4-(2-methylpiperidino)piperidine obtained according to a procedure disclosed in a literature *[Journal* of *Organic Chemistry,* vol. 55, p. 2552 (1990)] in a similar manner to Example 1.
$^1$H-NMR ($CDCl_3$) $\delta$ (ppm): 1.12 (d, J = 5.9 Hz, 3H), 1.29-1.77 (m, 10H), 2.13-2.20 (m, 1H), 2.53-2.61 (m, 1H), 2.68-2.88 (m, 3H), 2.96-3.13 (m, 1H), 4.62 (d, J = 5.9 Hz, 2H), 4.80-4.85 (m, 3H), 6.74-6.99 (m, 4H), 7.19-7.36 (m, 2H), 7.75 (s, 1H). APCIMS m/z: $[M + H]^+$ 514

Example 4

4-(2,4-difluorobenzylamino)-5-(2,4-difluorophenyl)-2-[4-(4-methylpiperidino)piperidino]pyrimidine (Compound 4)

Compound 4 (21 mg, 71%) was obtained using Compound A1 (21 mg, 0.057 mmol) obtained in Reference Example 1 and 4-(4-methylpiperidino)piperidine obtained according to a procedure disclosed in a literature *[Journal* of *Organic Chemistry,* vol. 55, p. 2552 (1990)] in a similar manner to Example 1.
$^1$H-NMR ($CDCl_3$) δ (ppm): 0.92 (d, J = 5.9 Hz, 3H), 1.19-1.53 (m, 5H), 1.63-1.67 (m, 2H), 1.85-1.90 (m, 2H), 2.14-2.22 (m, 2H), 2.53 (tt, J = 11.6, 3.6 Hz, 1H), 2.74-2.85 (m, 2H), 2.88-2.93 (m, 2H), 4.62 (d, J = 5.9 Hz, 2H), 4.77-4.85 (m, 3H), 6.73-6.99 (m, 4H), 7.18-7.35 (m, 2H), 7.75 (s, 1H). APCIMS m/z: $[M + H]^+$ 514

Example 5

4-(2,4-difluorobenzylamino)-5-(2,4-difluorophenyl)-2-[4-(cis-3,5-dimethylpiperidino)piperidino]pyrimidine (Compound 5)

Compound 5 (83 mg, 81%) was obtained using Compound A1 (71 mg, 0.193 mmol) obtained in Reference Example 1 and 4-(cis-3,5-dimethylpiperidino)piperidine obtained according to a procedure disclosed in a literature [*Journal. of Organic Chemistry,* vol. 55, p. 2552 (1990)] in a similar manner to Example 1.
$^1$H-NMR ($CDCl_3$) δ (ppm): 0.50-0.54 (m, 1H), 0.77-0.95 (m, 7H), 1.39-1.54 (m, 2H), 1.64-1.74 (m, 4H), 1.83-1.88 (m, 2H), 2.49-2.60 (m, 1H), 2.71-2.90 (m, 4H), 4.62 (d, J = 5.9 Hz, 2H), 4.78-4.86 (m, 3H), 6.74-6.99 (m, 4H), 7.15-7.35 (m, 2H), 7.75 (s, 1H).
APCIMS m/z: $[M + H]^+$ 528

Example 6

4-(2,4-difluorobenzylamino)-5-(2,4-difluorophenyl)-2-[4-(3-ethylpiperidino)piperidino]pyrimidine (Compound 6)

Compound 6 (18 mg, 47%) was obtained using Compound A1 (26 mg, 0.071 mmol) obtained in Reference Example 1 and 4-(3-ethylpiperidino)piperidine obtained according to a procedure disclosed in a literature *[Journal* of *Organic Chemistry,* vol. 55, p. 2552 (1990)] in a similar manner to Example 1.
$^1$H-NMR ($CDCl_3$) δ (ppm): 0.90 (t, J = 7.4 Hz, 3H), 1.17-1.28 (m, 2H), 1.45-2.04 (m,10H), 2.13-2.30 (m, 1H), 2.60-2.99 (m, 5H), 4.62 (d, J = 5.9 Hz, 2H), 4.81-4.85 (m, 3H), 6.74-6.99 (m, 4H), 7.19-7.35 (m, 2H), 7.75 (s, 1H).
APCIMS m/z: $[M + H]^+$ 528

Example 7

4-(2,4-difluorobenzylamino)-5-(2,4-difluorophenyl)-2-[4-(3-hydroxymethylpiperidino)piperidino]pyrimidine (Compound 7)

Compound 7 (17 mg, 54%) was obtained using Compound A1 (22 mg, 0.060 mmol) obtained in Reference Example 1 and 4-(3-hydroxymethylpiperidino)piperidine obtained according to a procedure disclosed in a literature *[Journal* of *Organic Chemistry,* vol. 55, p. 2552 (1990)] in a similar manner to Example 1.
$^1$H-NMR ($CDCl_3$) δ (ppm): 1.42-1.87 (m, 10H), 2.25-2.95 (m, 6H), 3.56 (dd, J = 10.6, 5.8 Hz, 2H), 3.69 (dd, J = 10.6, 4.9 Hz, 1H), 4.62 (d, J = 5.9 Hz, 2H), 4.78-4.86 (m, 3H), 6.74-6.99 (m, 4H), 7.19-7.35 (m, 2H), 7.75 (s, 1H).
APCIMS m/z: $[M + H]^+$ 530

Example 8

2-[4-(3-carbamoylpiperidino)piperidino]-4-(2,4-difluorobenzylamino)-5-(2,4-difluorophenyl)pyrimidine (Compound 8)

Compound 8 (18 mg, 57%) was obtained using Compound A1 (22 mg, 0.060 mmol) obtained in Reference Example 1 and 4-(3-carbamoylpiperidino)piperidine obtained according to a procedure disclosed in a literature *[Journal of Organic Chemistry,* vol. 55, p. 2552 (1990)] in a similar manner to Example 1.
$^1$H-NMR ($CDCl_3$) δ (ppm): 1.32-1.85 (m, 8H), 2.35-2.69 (m, 4H), 2.70-3.00 (m, 4H), 4.62 (d, J = 5.9 Hz, 2H), 4.79-4.89 (m, 3H), 5.47-5.66 (m, 1H), 6.74-6.99 (m, 4H), 7.19-7.34 (m, 2H), 7.75 (s, 1H).
APCIMS m/z: $[M + H]^+$ 543

Example 9

4-(2,4-difluorobenzylamino)-5-(2,4-difluorophenyl)-2-[4-(3-ethoxycarbonylpiperidino)piperidino]pyrimidine (Compound 9)

Compound 9 (18 mg, 53%) was obtained using Compound A1 (22 mg, 0.060 mmol) obtained in Reference Example 1 and 4-(3-ethoxycarbonylpiperidino)piperidine obtained according to a procedure disclosed in a literature *[Journal* of *Organic Chemistry,* vol. 55, p. 2552 (1990)] in a similar manner to Example 1.
$^1$H-NMR ($CDCl_3$) δ (ppm): 1.26 (t, J = 7.2 Hz, 3H), 1.39-1.98 (m, 8H), 2.24 (td, J = 10.8, 2.7 Hz, 1H), 2.39 (t, J = 10.5 Hz, 1H), 2.52-2.63 (m, 2H), 2.76-2.84 (m, 3H), 3.00-3.04 (m, 1H), 4.13 (q, J = 7.1 Hz, 2H), 4.62 (d, J = 5.8 Hz, 2H), 4.77-4.86 (m, 3H), 6.74-6.99 (m, 4H), 7.19-7.35 (m, 2H), 7.75 (s, 1H).
APCIMS m/z: $[M + H]^+$ 572

Example 10

2-[4-(N-butyl-N-methylamino)piperidino]-4-(2,4-difluorobenzylamino)-5-(2,4-difluorophenyl)pyrimidine (Compound 10)

Compound 10 (25 mg, 71%) was obtained using Compound A1 (26 mg, 0.071 mmol) obtained in Reference Example 1 and 4-(N-butyl-N-methylamino)piperidine obtained according to a procedure disclosed in a literature *[Journal of Organic Chemistry,* vol. 55, p. 2552 (1990)] in a similar manner to Example 1.
$^1$H-NMR ($CDCl_3$) δ (ppm): 0.94 (t, J = 7.3 Hz, 3H), 1.26-1.58 (m, 6H), 1.78-1.93 (m, 2H), 2.32 (s, 3H), 2.48-2.54 (m, 2H), 2.69-2.85 (m, 3H), 4.62 (d, J = 5.8 Hz, 2H), 4.81-4.85 (m, 3H), 6.74-6.98 (m, 4H), 7.19-7.35 (m, 2H), 7.75 (s, 1H).
APCIMS m/z: $[M + H]^+$ 502

Example 11

4-(2,4-difluorobenzylamino)-5-(2-fluorophenyl)-2-[4-(3-methylpiperidino)pyrimidino]pyrimidine (Compound 11)

Compound 11 (0.102 g, 78%) was obtained using Compound A2 (93 mg) obtained in Reference Example 2 and 4-(3-methylpiperidino)pyrimidine obtained according to a procedure disclosed in a literature *[Journal of Organic Chemistry,* vol. 55, p. 2552 (1990)] in a similar manner to Example 1.
$^1$H-NMR ($CDCl_3$) δ (ppm): 0.86 (d, J = 6.3 Hz, 3H), 1.38-1.88 (m, 10H), 2.15 (td, J = 11.1, 3.1 Hz, 1H), 2.53 (tt, J = 11.5, 3.0 Hz, 1H), 2.74-2.89 (m, 4H), 4.63 (d, J = 5.9 Hz, 2H), 4.80 (d, J = 13.3 Hz, 2H), 4.95-5.00 (m, 1H), 6.73-6.84 (m, 2H), 7.11-7.37 (m, 5H), 7.79 (s, 1H).
APCIMS m/z: $[M + H]^+$ 496

Example 12

4-(2,4-difluorobenzylamino)-2-[4-(cis-3,5-dimethylpiperidino)piperidino]-5-(2-fluorophenyl)pyrimidine (Compound 12)

Compound 12 (13 mg, 85%) was obtained using Compound A2 (20 mg) obtained in Reference Example 2 and 4-(cis-3,5-dimethylpiperidino)piperidine obtained according to a procedure disclosed in a literature *[Journal of Organic Chemistry,* vol. 55, p. 2552 (1990)] in a similar manner to Example 1.
$^1$H-NMR ($CDCl_3$) δ (ppm) : 0.45-0.65 (m, 1H), 0.86 (d, J = 5.8 Hz, 6H), 1.40-1.89 (m, 9H), 2.58 (tt, J = 11.6, 3.4 Hz, 1H), 2.74-2.86 (m, 4H), 4.63 (d, J = 5.9 Hz, 2H), 4.81 (d, J = 13.2 Hz, 2H), 4.93 (t, J = 6.0 Hz, 1H), 6.74-6.85 (m, 2H), 7.11-7.39 (m, 5H), 7.80 (s, 1H).
APCIMS m/z: $[M + H]^+$ 510

Example 13

4-(2,4-dichlorobenzylamino)-5-(2,4-difluorophenyl)-2-[4-(3-methylpiperidino)piperidino]pyrimidine (Compound 13)

Compound 13 (10 mg, 29%) was obtained using Compound A3 (25 mg) obtained in Reference Example 3 and 4-(3-methylpiperidino)piperidine obtained according to a procedure disclosed in a literature *[Journal* of *Organic Chemistry,* vol. 55, p. 2552 (1990)] in a similar manner to Example 1.
$^1$H-NMR ($CDCl_3$) δ (ppm): 0.88 (d, J = 5.9 Hz, 3H), 1.39-1.92 (m, 10H), 2.10-2.23 (m, 1H), 2.60-2.97 (m, 5H), 4.64 (d, J = 5.9 Hz, 2H), 4.77 (d, J = 13.3 Hz, 2H), 4.96 (t, J = 5.9 Hz, 1H), 6.88-6.99 (m, 2H), 7.16-7.31 (m, 3H), 7.37 (d, J =

2.0 Hz, 1H), 7.76 (s, 1H).
APCIMS m/z: $[M + H]^+$ 546

Example 14

4-(2,4-dichlorobenzylamino)-5-(2,4-difluorophenyl)-2-[4-(2-piperidinoethyl)piperazin-1-yl]pyrimidine (Compound 14)

Compound 14 (16 mg, 44%) was obtained using Compound A3 (26 mg) obtained in Reference Example 3 and 1-(2-piperidinoethyl)piperazine in a similar manner to Example 1. $^1$H-NMR ($CDCl_3$) δ (ppm): 1.42-1.64 (m, 6H), 2.43-2.59 (m, 12H), 3.75-3.79 (m, 4H), 4.65 (d, J = 6.1 Hz, 2H), 4.95 (t, J = 5.8 Hz, 1H), 6.88-7.00 (m, 2H), 7.16-7.36 (m, 4H), 7.76 (s, 1H).
APCIMS m/z: $[M + H]^+$ 561

Example 15

4-(2,6-difluorobenzylamino)-5-(2,4-difluorophenyl)-2-[4-(3-methylpiperidino)piperidino]pyrimidine (Compound 15)

Compound B1 (64 mg, 0.13 mmol) obtained in Reference Example 4 was dissolved in degassed toluene (0.51 mL) under argon atmosphere, and the solution was added with tetrakis(triphenylphosphine)palladium (0) (24 mg, 0.020 mmol), dihydroxy(2,4-difluorophenyl)borane (32 mg, 0.20 mmol) and a 1 mol/L aqueous sodium hydrogencarbonate solution (0.33 mL), followed by stirring at 100°C for 9 hours. Water was added to the reaction mixture, followed by extraction with ethyl acetate. The organic layer was washed with saturated brine and then dried over anhydrous sodium sulfate, followed by evaporation of the solvent. The residue was purified by preparative thin-layer chromatography (ethyl acetate : hexane : triethylamine = 10:90:10) to obtain Compound 15 (9.4 mg, 14%).
$^1$H NMR ($CDCl_3$) δ (ppm): 0.88 (d, J = 6.1 Hz, 3H), 1.46-1.98 (m, 10H), 2.14-2.25 (m, 1H), 2.65-2.99 (m, 5H), 4.71 (d, J = 6.1 Hz, 2H), 4.90-4.95 (m, 3H), 6.81-6.98 (m, 4H), 7.15-7.26 (m, 2H), 7.72 (s, 1H).
APCIMS m/z: $[M + H]^+$ 514

Example 16

4-(2,6-difluorobenzylamino)-5-(2,4-difluorophenyl)-2-[4-(cis-3,5-dimethylpiperidino)piperidino]pyrimidine (Compound 16)

Compound 16 (18 mg, 28%) was obtained using Compound B3 (72 mg) obtained in Reference Example 6 and dihydroxy(2,4-difluorophenyl)borane in a similar manner to Example 15. $^1$H-NMR ($CDCl_3$) δ (ppm): 0.43-0.65 (m, 1H), 0.86 (d, J = 5.8 Hz, 6H), 1.43-1.92 (m, 9H), 2.52-2.70 (m, 1H), 2.76-2.87 (m, 4H), 4.71 (d, J = 6.1 Hz, 2H), 4.88-4.93 (m, 3H), 6.83-6.98 (m, 4H), 7.16-7.23 (m, 2H), 7.72 (s, 1H).
APCIMS m/z: $[M + H]^+$ 528

Example 17

4-(2,6-difluorobenzylamino)-2-[4-(cis-3,5-dimethylpiperidino)piperidino]-5-(2-fluorophenyl)pyrimidine (Compound 17)

Compound 17 (38 mg, 44%) was obtained using Compound B3 (91 mg) obtained in Reference Example 6 and dihydroxy(2-fluorophenyl)borane in a similar manner to Example 15. $^1$H-NMR ($CDCl_3$) δ (ppm): 0.45-0.72 (m, 1H), 0.75-0.96 (m, 7H), 1.45-2.03 (m, 8H), 2.60-2.97 (m, 5H), 4.72 (d, J = 5.9 Hz, 2H), 4.93-5.06 (m, 3H), 6.83-6.89 (m, 2H), 7.11-7.36 (m, 5H), 7.77 (s, 1H).
APCIMS m/z: $[M + H]^+$ 510

Example 18

4-(2,6-difluorobenzylamino)-5-(2-naphtyl)-2-(4-piperidinopiperidino)pyrimidine (Compound 18)

Compound 18 (21 mg, 37%) was obtained using Compound B2 (50 mg) obtained in Reference Example 5 and dihydroxy(2-naphthyl)borane in a similar manner to Example 15.
$^1$H-NMR ($CDCl_3$) δ (ppm): 1.43-1.65 (m, 8H), 1.87-1.92 (m, 2H), 2.50-2.56 (m, 5H), 2.82 (dt, J = 2.2, 13.0 Hz, 2H), 4.72 (d, J = 6.2 Hz, 2H), 4.90-4.95 (m, 2H), 5.43 (t, J = 5.9 Hz, 1H), 6.86 (t, J = 8.4 Hz, 2H), 7.14-7.23 (m, 1H), 7.42 (dd, J = 2.1, 5.9 Hz, 1H), 7.45-7.53 (m, 2H), 7.76-7.90 (m, 4H). APCIMS m/z: $[M + H]^+$ 514

Example 19

4-(2,6-difluorobenzylamino)-5-(1-naphtyl)-2-(4-piperidinopiperidino)pyrimidine (Compound 19)

Compound 19 (35 mg, 63%) was obtained using Compound B2 (50 mg) obtained in Reference Example 5 and dihydroxy(1-naphthyl)borane in a similar manner to Example 15. $^1$H-NMR ($CDCl_3$) δ (ppm): 1.46-1.60 (m, 8H), 1.89-1.94 (m, 2H), 2.54-2.58 (m, 5H), 2.84 (dt, J = 3.2, 10.8 Hz, 2H), 4.63 (d, J = 5.9 Hz, 2H), 4.77 (t, J = 5.7 Hz, 1H), 4.92-4.97 (m, 2H), 6.78 (t, J = 8.1 Hz, 2H), 7.11-7.22. (m, 1H), 7.34-7.40 (m, 2H), 7.46-7.54 (m, 2H), 7.67 (d, J = 8.5 Hz, 1H), 7.82 (s, 1H), 7.87 (t, J = 6.8 Hz, 2H).
APCIMS m/z: $[M + H]^+$ 514

Example 20

5-(2-biphenyl)-4-(2,6-difluorobenzylamino)-2-(4-piperidinopiperidino)pyrimidine (Compound 20)

Compound 20 (16 mg, 22%) was obtained using Compound B2 (63 mg) obtained in Reference Example 5 and dihydroxy(2-biphenyl)borane in a similar manner to Example 15.
$^1$H-NMR ($CDCl_3$) δ (ppm): 1.41-1.78 (m, 8H), 1.82-1.98 (m, 2H), 2.45-2.80 (m, 7H), 4.41 (dd, J = 14.6, 6.3 Hz, 1H), 4.61 (dd, J = 14.6, 5.9 Hz, 1H), 4.72-4.84 (m, 3H), 6.77-6.87 (m, 2H), 7.13-7.27 (m, 7H), 7.35-7.43 (m, 3H), 7.59 (s, 1H).
APCIMS m/z: $[M + H]^+$ 540

Example 21

5-(3-biphenyl)-4-(2,6-difluorobenzylamino)-2-(4-piperidinopiperidino)pyrimidine (Compound 21)

Compound 21 (29 mg, 48%) was obtained using Compound B2 (50 mg) obtained in Reference Example 5 and dihydroxy(3-biphenyl)borane in a similar manner to Example 15.
$^1$H-NMR ($CDCl_3$) δ (ppm): 1.42-1.64 (m, 8H), 1.87-1.91 (m, 2H), 2.52-2.55 (m, 5H), 2.81 (dt, J = 2.2, 13.0 Hz, 2H), 4.73 (d, J = 6.2 Hz, 2H), 4.88-4.93 (m, 2H), 5.42 (t, J = 5.9 Hz, 1H), 6.86 (t, J = 7.8 Hz, 2H), 7.15-7.60 (m, 10H), 7.83 (s, 1H). APCIMS m/z: $[M + H]^+$ 540

Example 22

5-(4-biphenyl)-4-(2,6-difluorobenzylamino)-2-(4-piperidinopiperidino)pyrimidine (Compound 22)

Compound 22 (36 mg, 61%) was obtained using Compound B2 (50 mg) obtained in Reference Example 5 and dihydroxy(4-biphenyl)borane in a similar manner to Example 15.
$^1$H-NMR ($CDCl_3$) δ (ppm): 1.45-1.62 (m, 8H), 1.87-1.91 (m, 2H), 2.52-2.56 (m, 5H), 2.82 (dt, J = 1.4, 10.8 Hz, 2H), 4.73 (d, J = 6.2 Hz, 2H), 4.89-4.93 (m, 2H), 5.41 (t, J = 5.9 Hz, 1H), 6.87 (t, J = 7.8 Hz, 2H), 7.17-7.24 (m, 1H), 7.25-7.48 (m, 5H), 7.63 (dt, J = 1.9, 6.8 Hz, 4H), 7.83 (s, 1H). APCIMS m/z: $[M + H]^+$ 540

Example 23

4-(2,6-difluorobenzylamino)-2-(4-piperidinopiperidino)-5-(2-thienyl)pyrimidine (Compound 23)

Compound 23 (25 mg, 53%) was obtained using Compound B2 (50 mg) obtained in Reference Example 5 and dihydroxy(2-thienyl)borane in a similar manner to Example 15.
$^1$H-NMR ($CDCl_3$) δ (ppm): 1.41-1.60 (m, 8H), 1.85-1.90 (m, 2H), 2.51-2.55 (m, 5H), 2.80 (dt, J = 3.2, 10.8 Hz, 2H), 4.73 (d, J = 6.2 Hz, 2H), 4.87-4.92 (m, 2H), 5.66 (t, J = 5.9 Hz, 1H), 6.87 (t, J = 7.8 Hz, 2H), 6.98 (dd, J = 0.8, 3.2 Hz, 1H), 7.09 (dd, J = 3.5, 5.1 Hz, 1H), 7.15-7.24 (m, 1H), 7.29 (dd, J = 1.1, 5.1 Hz, 1H), 7.88 (s, 1H).
APCIMS m/z: $[M + H]^+$ 470

Example 24

4-(2,6-difluorobenzylamino)-5-(5-methyl-2-thienyl)-2-(4-piperidinopiperidino)pyrimidine (Compound 24)

Compound 24 (23 mg, 48%) was obtained using Compound B2 (50 mg) obtained in Reference Example 5 and dihydroxy(5-methyl-2-thienyl)borane in a similar manner to Example 15. $^1$H-NMR ($CDCl_3$) δ (ppm): 1.44-1.59 (m,

8H), 1.84-1.89 (m, 2H), 2.48 (s, 3H), 2.50-2.52 (m, 5H), 2.79 (dt, J = 3.2, 12.1 Hz, 2H), 4.72 (d, J = 5.9 Hz, 2H), 4.86-4.91 (m, 2H), 5.68 (t, J = 7.8 Hz, 1H), 6.73 (m, 2H), 6.86 (t, J = 7.8 Hz, 2H), 7.20 (t, J = 8.1 Hz, 1H), 7.84 (s, 1H).
APCIMS m/z: $[M + H]^+$ 484

Example 25

4-(2,6-difluorobenzylamino)-2-(4-piperidinopiperidino)-5-(3-thienyl)pyrimidine (Compound 25)

Compound 25 (21 mg, 45%) was obtained using Compound B2 (50 mg) obtained in Reference Example 5 and dihydroxy(3-thienyl)borane in a similar manner to Example 15.
$^1$H-NMR ($CDCl_3$) δ (ppm): 1.42-1.64 (m, 8H), 1.85-1.90 (m, 2H), 2.47-2.55 (m, 5H), 2.79 (dt, J = 3.2, 13.0 Hz, 2H), 4.73 (d, J = 5.9 Hz, 2H), 4.86-4.91 (m, 2H), 5.43 (t, J = 5.9 Hz, 1H), 6.87 (t, J = 8.1 Hz, 2H), 7.09 (dd, J = 1.1, 5.1 Hz, 1H), 7.17-7.24 (m, 2H), 7.42 (dd, J = 3.0, 5.1 Hz, 1H), 7.83 (s, 1H).
APCIMS m/z: $[M + H]^+$ 470

Example 26

4-(2,6-difluorobenzylamino)-5-(2-methylphenyl)-2-(4-piperidinopiperidino)pyrimidine (Compound 26)

Compound 26 (20 mg, 42%) was obtained using Compound B2 (50 mg) obtained in Reference Example 5 and dihydroxy(2-methylphenyl)borane in a similar manner to Example 15.
$^1$H-NMR ($CDCl_3$) δ (ppm): 1.45-1.65 (m, 8H), 1.87-1.92 (m, 2H), 2.12 (s, 3H), 2.53-2.56 (m, 5H), 2.81 (dt, J = 2.2, 13.2 Hz, 2H), 4.64-4.72 (m, 3H), 4.87-4.92 (m, 2H), 6.83 (t, J = 8.1 Hz, 2H), 7.09-7.28 (m, 5H), 7.66 (s, 1H).
APCIMS m/z: $[M + H]^+$ 478

Example 27

4-(2,6-difluorobenzylamino)-5-(3-methylphenyl)-2-(4-piperidinopiperidino)pyrimidine (Compound 27)

Compound 27 (28 mg, 54%) was obtained using Compound B2 (50 mg) obtained in Reference Example 5 and dihydroxy(3-methylphenyl)borane in a similar manner to Example 15.
$^1$H-NMR ($CDCl_3$) δ (ppm): 1.44-1.64 (m, 8H), 1.85-1.90 (m, 2H), 2.38 (s, 3H), 2.51-2.55 (m, 5H), 2.80 (dt, J = 2.2, 13.0 Hz, 2H), 4.70 (d, J = 5.9 Hz, 2H), 4.86-4.91 (m, 2H), 5.38 (t, J = 7.8 Hz, 1H), 6.86 (t, J = 8.1 Hz, 2H), 7.08-7.33 (m, 5H), 7.76 (s, 1H).
APCIMS m/z: $[M + H]^+$ 478

Example 28

4-(2,6-difluorobenzylamino)-5-(4-methylphenyl)-2-(4-piperidinopiperidino)pyrimidine (Compound 28)

Compound 28 (23 mg, 44%) was obtained using Compound B2 (50 mg) obtained in Reference Example 5 and dihydroxy(4-methylphenyl)borane in a similar manner to Example 15.
$^1$H-NMR ($CDCl_3$) δ (ppm): 1.45-1.59 (m, 8H), 1.85-1.90 (m, 2H), 2.38 (s, 3H), 2.51-2.55 (m, 5H), 2.79 (dt, J = 3.2, 10.8 Hz, 2H), 4.70 (d, J = 5.9 Hz, 2H), 4.86-4.91 (m, 2H), 5.33 (t, J = 7.8 Hz, 1H), 6.85 (t, J = 8.1 Hz, 2H), 7.16-7.24 (m, 5H), 7.75 (s, 1H).
APCIMS m/z: $[M + H]^+$ 478

Example 29

4-(2,6-difluorobenzylamino)-5-(2-fluorophenyl)-2-(4-piperidinopiperidino)pyrimidine (Compound 29)

Compound 29 (61 mg, 83%) was obtained using Compound B2 (71 mg) obtained in Reference Example 5 and dihydroxy(2-fluorophenyl)borane in a similar manner to Example 15.
$^1$H-NMR ($CDCl_3$) δ (ppm): 1.47-1.80 (m, 8H), 1.95 (d, J = 11.9 Hz, 2H), 2.52-2.86 (m, 7H), 4.72 (d, J = 5.9 Hz, 2H), 4.93 (d, J = 13.4 Hz, 2H), 5.02 (t, J = 5.9 Hz, 1H), 6.80-6.90 (m, 2H), 7.08-7.36 (m, 5H), 7.77 (s, 1H).
APCIMS m/z: $[M + H]^+$ 482

Example 30

4-(2,6-difluorobenzylamino)-5-(3-fluorophenyl)-2-(4-piperidinopiperidino)pyrimidine (Compound 30)

Compound 30 (32 mg, 60%) was obtained using Compound B2 (50 mg) obtained in Reference Example 5 and dihydroxy(3-fluorophenyl)borane in a similar manner to Example 15.
$^1$H-NMR ($CDCl_3$) δ (ppm): 1.42-1.64 (m, 8H), 1.86-1.91 (m, 2H), 2.51-2.55 (m, 5H), 2.81 (dt, J = 2.2, 13.2 Hz, 2H), 4.72 (d, J = 6.2 Hz, 2H), 4.87-4.91 (m, 2H), 5.33 (t, J = 5.9 Hz, 1H), 6.87 (t, J = 8.1 Hz, 2H), 6.98-7.10 (m, 3H), 7.15-7.24 (m, 1H), 7.34-7.42 (m, 1H), 7.77 (s, 1H).
APCIMS m/z: $[M + H]^+$ 482

Example 31

4-(2,6-difluorobenzylamino)-5-(4-fluorophenyl)-2-(4-piperidinopiperidino)pyrimidine (Compound 31)

Compound 31 (36 mg, 67%) was obtained using Compound B2 (50 mg) obtained in Reference Example 5 and dihydroxy(4-fluorophenyl)borane in a similar manner to Example 15.
$^1$H-NMR ($CDCl_3$) δ (ppm): 1.42-1.64 (m, 8H), 1.86-1.91 (m, 2H), 2.51-2.55 (m, 5H), 2.80 (dt, J = 2.2, 13.2 Hz, 2H), 4.70 (d, J = 6.2 Hz, 2H), 4.86-4.91 (m, 2H), 5.20 (t, J = 5.9 Hz, 1H), 6.86 (t, J = 8.1 Hz, 2H), 7.10-7.28 (m, 5H), 7.72 (s, 1H).
APCIMS m/z: $[M + H]^+$ 482

Example 32

5-(2-chlorophenyl)-4-(2,6-difluorobenzylamino)-2-(4-piperidinopiperidino)pyrimidine (Compound 32)

Compound 32 (37mg, 49%) was obtained using Compound B2 (78 mg) obtained in Reference Example 5 and dihydroxy(2-chlorophenyl)borane in a similar manner to Example 15.
$^1$H-NMR ($CDCl_3$) δ (ppm): 1.46-1.69 (m, 8H), 1.80-2.03 (m, 2H), 2.57-2.64 (m, 5H), 2.76-2.87 (m, 2H), 4.65-4.80 (m, 3H), 4.93 (d, J = 13.2 Hz, 2H), 6.78-6.89 (m, 2H), 7.12-7.33 (m, 4H), 7.43-7.51 (m, 1H), 7.71 (s, 1H).
APCIMS m/z: $[M + H]^+$ 498

Example 33

5-(3-chlorophenyl)-4-(2,6-difluorobenzylamino)-2-(4-piperidinopiperidino)pyrimidine (Compound 33)

Compound 33 (12 mg, 15%) was obtained using Compound B2 (75 mg) obtained in Reference Example 5 and dihydroxy(3-chlorophenyl)borane in a similar manner to Example 15.
$^1$H-NMR ($CDCl_3$) δ (ppm): 1.41-1.80 (m, 8H), 1.85-2.10 (d, J = 12.2 Hz, 2H), 2.50-2.85 (m, 7H), 4.71 (d, J = 5.9 Hz, 2H), 4.92 (d, J = 13.4 Hz, 2H), 5.30 (t, J = 5.9 Hz, 1H), 6.82-6.92 (m, 2H), 7.16-7.38 (m, 5H), 7.74 (s, 1H).
APCIMS m/z: $[M + H]^+$ 498

Example 34

5-(4-chlorophenyl)-4-(2,6-difluorobenzylamino)-2-(4-piperidinopiperidino)pyrimidine (Compound 34)

Compound 34 (10 mg, 9%) was obtained using Compound B2 (61 mg) obtained in Reference Example 5 and dihydroxy(4-chlorophenyl)borane in a similar manner to Example 15.
$^1$H-NMR ($CDCl_3$) δ (ppm): 1.41-1.80 (m, 8H), 1.85-2.07 (m, 2H), 2.45-2.85 (m, 7H), 4.70 (d, J = 5.9 Hz, 2H), 4.91 (d, J = 13.2 Hz, 2H), 5.23 (t, J = 5.9 Hz, 1H), 6.82-6.91 (m, 2H), 7.16-7.26 (m, 3H), 7.31-7.40 (m, 2H), 7.73 (s, 1H).
APCIMS m/z: $[M + H]^+$ 498

Example 35

4-(2,6-difluorobenzylamino)-5-(2-methoxyphenyl)-2-(4-piperidinopiperidino)pyrimidine (Compound 35)

Compound 35 (7 mg, 9%) was obtained using Compound B2 (71 mg) obtained in Reference Example 5 and dihydroxy(2-methoxyphenyl)borane in a similar manner to Example 15.
$^1$H-NMR ($CDCl_3$) δ (ppm): 1.40-1.85 (m, 8H), 1.96-2.01 (m, 2H), 2.50-2.85 (m, 7H), 3.72 (s, 3H), 4.58-4.83 (m, 2H),

4.95 (d, J = 13.5 Hz, 2H), 5.09 (t, J = 6.2 Hz, 1H), 6.83-7.04 (m, 4H), 7.14-7.36 (m, 3H), 7.75 (s, 1H).
APCIMS m/z: $[M + H]^+$ 494

Example 36

4-(2,6-difluorobenzylamino)-5-(3-methoxyphenyl)-2-(4-piperidinopiperidino)pyrimidine (Compound 36)

Compound 36 (9.3 mg, 14%) was obtained using Compound B2 (63 mg) obtained in Reference Example 5 and dihydroxy(3-methoxyphenyl)borane in a similar manner to Example 15.
$^1$H-NMR ($CDCl_3$) δ (ppm): 1.41-1.80 (m, 8H), 1.85-2.05 (m, 2H), 2.45-2.90 (m, 7H), 3.81 (s, 3H), 4.70 (d, J = 5.9 Hz, 2H), 4.91 (d, J = 13.4 Hz, 2H), 5.43 (t, J = 5.9 Hz, 1H), 6.81-6.91 (m, 5H), 7.15-7.36 (m, 2H), 7.78(s, 1H).
APCIMS m/z: $[M + H]^+$ 494

Example 37

4-(2,6-difluorobenzylamino)-5-(4-methoxyphenyl)-2-(4-piperidinopiperidino)pyrimidine (Compound 37)

Compound 37 (9.4 mg, 15%) was obtained using Compound B2 (61 mg) obtained in Reference Example 5 and dihydroxy(4-methoxyphenyl)borane in a similar manner to Example 15.
$^1$H-NMR ($CDCl_3$) δ (ppm): 1.37-1.72 (m, 8H), 1.77-1.96 (m, 2H), 2.45-2.66 (m, 5H), 2.75-2.84 (m, 2H), 3.83 (s, 3H), 4.70 (d, J = 6.2 Hz, 2H), 4.90 (d, J = 13.2 Hz,2H), 5.28 (t, J = 6.1 Hz, 1H), 6.81-6.98 (m, 4H), 7.14-7.24 (m, 3H), 7.73 (s, 1H).
APCIMS m/z: $[M + H]^+$ 494

Example 38

4-(2,6-difluorobenzylamino)-2-(4-piperidinopiperidino)-5-(3-pyridyl)pyrimidine (Compound 38)

Compound 38 (17 mg, 33%) was obtained using Compound B2 (50 mg) obtained in Reference Example 5 and dihydroxy(3-pyridyl)borane in a similar manner to Example 15.
$^1$H-NMR ($CDCl_3$) δ (ppm): 1.47-1.60 (m, 8H), 1.87-1.91 (m, 2H), 2.51-2.55 (m, 5H), 2.81 (dt, J = 3.2, 10.8 Hz, 2H), 4.72 (d, J = 5.9 Hz, 2H), 4.87-4.92 (m, 2H), 5.16 (t, J = 7.8 Hz, 1H), 6.86 (t, J = 8.1 Hz, 2H), 7.16-7.24 (m, 5H), 7.75 (s, 1H).
APCIMS m/z: $[M + H]^+$ 465

Example 39

5-(2-benzofuranyl)-4-(2,6-difluorobenzylamino)-2-(4-piperidinopiperidino)pyrimidine (Compound 39)

Compound 39 (5.9 mg, 10%) was obtained using Compound B2 (55 mg) obtained in Reference Example 5 and dihydroxy(2-benzofuranyl)borane in a similar manner to Example 15.
$^1$H-NMR ($CDCl_3$) δ (ppm): 1.38-1.80 (m, 8H), 1.80-1.97 (m, 2H), 2.41-2.63 (m, 5H), 2.79-2.88 (m, 2H), 4.86 (d, J = 6.1 Hz, 2H), 4.93 (brd, J = 13.7 Hz, 2H), 6.64-6.68 (m, 2H), 6.86-6.93 (m, 2H), 7.19-7.32 (m, 3H), 7.46-7.54 (m, 2H), 8.26 (s, 1H).
APCIMS m/z: $[M + H]^+$ 504

Example 40

4-(2,6-difluorobenzylamino)-5-(2,4-difluorophenyl)-2-(4-piperidinopiperidino)pyrimidine (Compound 40)

Compound 40 (28 mg, 50%) was obtained using Compound B2 (50 mg) obtained in Reference Example 5 and dihydroxy(2,4-difluorophenyl)borane in a similar manner to Example 15.
$^1$H-NMR ($CDCl_3$) δ (ppm): 1.45-1.64 (m, 8H), 1.86-1.91 (m, 2H), 2.51-2.55 (m, 5H),2.81 (dt, J = 2.4, 13.2 Hz, 2H), 4.72 (d, J = 5.9 Hz, 2H), 4.87-4.92 (m, 3H), 6.82-6.98 (m, 4H), 7.16-7.25 (m, 2H), 7.73 (s, 1H).
APCIMS m/z: $[M + H]^+$ 500

Example 41

4-(2,6-difluorobenzylamino)-5-(2,5-difluorophenyl)-2-(4-piperidinopiperidino)pyrimidine (Compound 41)

Compound 41 (24 mg, 43%) was obtained using Compound B2 (50 mg) obtained in Reference Example 5 and dihydroxy(2,5-difluorophenyl)borane in a similar manner to Example 15.
$^1$H-NMR ($CDCl_3$) δ (ppm): 1.44-1.64 (m, 8H), 1.86-1.91 (m, 2H), 2.51-2.55 (m, 5H), 2.81 (dt, J = 2.2, 13.2 Hz, 2H), 4.73 (d, J = 5.9 Hz, 2H), 4.88-4.93 (m, 2H), 4.99 (t, J = 5.4 Hz, 1H), 6.86 (t, J = 8.1 Hz, 2H), 6.92-7.24 (m, 4H), 7.77 (s, 1H).
APCIMS m/z: $[M + H]^+$ 500

Example 42

4-(2,6-difluorobenzylamino)-2-(4-piperidinopiperidino)-5-[3-(trifluoromethyl)phenyl]pyrimidine (Compound 42)

Compound 42 (45 mg, 65%) was obtained using Compound B2 (60 mg) obtained in Reference Example 5 and dihydroxy[3-(trifluoromethyl)phenyl]borane in a similar manner to Example 15.
$^1$H-NMR ($CDCl_3$) δ (ppm): 1.35-1.75 (m, 8H), 1.80-1.92 (m, 2H), 2.40-2.63 (m, 5H), 2.81 (dt, J = 2.0, 12.0 Hz, 2H), 4.72 (d, J = 5.9 Hz, 2H), 4.81-4.95 (m, 2H), 5.23 (t, J = 5.9 Hz, 1H), 6.80-6.94 (m, 2H), 7.12-7.30 (m, 1H), 7.42-7.63 (m, 4H), 7.77 (s, 1H).
APCIMS m/z: $[M + H]^+$ 532

Example 43

5-(3-cyanophenyl)-4-(2,6-difluorobenzylamino)-2-(4-piperidinopiperidino)pyrimidine (Compound 43)

Compound 43 (13 mg, 17%) was obtained using Compound B2 (81 mg) obtained in Reference Example 5 and dihydroxy(3-cyanophenyl)borane in a similar manner to Example 15.
$^1$H-NMR ($CDCl_3$) δ (ppm): 1.45-1.60 (m, 4H), 1.71-1.75 (m, 4H), 1.97-2.03 (m, 2H), 2.67-2.87 (m, 7H), 4.72 (d, J = 6.1 Hz, 2H), 4.93 (d, J = 13.2 Hz, 2H), 5.15-5.20 (m, 1H), 6.83-6.93 (m, 2H), 7.17-7.28 (m, 1H), 7.52-7.64 (m, 4H), 7.77 (m, 1H). APCIMS m/z: $[M + H]^+$ 489

Example 44

4-(2,6-difluorobenzylamino)-5-(2-furyl)-2-(4-piperidinopiperidino)pyrimidine (Compound 44)

Compound B2 (72 mg, 0.15 mmol) obtained in Reference Example 5 was dissolved in degassed THF (0.6 mL) under argon atmosphere, and the solution was added with dichlorobis(triphenylphosphine)palladium (II) (6.4 mg, 7.7 μmol) and 2-(tributylstannyl)furan (58 μL, 0.19 mmol), followed by stirring at 80°C for 6 hours. After the reaction mixture was cooled, a saturated aqueous potassium fluoride solution and diethyl ether were added to the reaction mixture, followed by stirring for 30 minutes. The resulting mixture was subjected to vacuum filtration, whereby a solid was removed from the mixture. The filtrate was extracted with diethyl ether. The organic layer was washed with saturated brine and then dried over anhydrous sodium sulfate, followed by evaporation of the solvent. The residue was purified by preparative thin-layer chromatography (ethyl acetate : hexane : triethylamine = 25:75:10) to obtain Compound 44 (23 mg, 32%).
$^1$H NMR ($CDCl_3$) δ (ppm): 1.35-1.72 (m, 8H), 1.77-1.95 (m, 2H), 2.40-2.67 (m, 5H), 2.70-2.93 (m, 2H), 4.81 (d, J = 5.9 Hz, 2H), 4.87-4.92 (m, 2H), 6.33 (dd, J = 3.6, 0.7 Hz, 1H), 6.38 (t, J = 5.9 Hz, 1H), 6.44 (dd, J = 3.3, 2.0 Hz, 1H), 6.78-6.93 (m, 2H), 7.10-7.28 (m, 1H), 7.42-7.47 (m, 1H), 8.08 (s, 1H).
APCIMS m/z: $[M + H]^+$ 454

Example 45

4-(2,6-difluorobenzylamino)-5-(2-hydroxyphenyl)-2-(4-piperidinopiperidino)pyrimidine (Compound 45)

A 1.0 mol/L solution (0.51 mL, 0.51 mmol) of boron tribromide in dichloromethane was added to Compound 35 obtained in Example 35 (31 mg, 0.064 mmol) at 0°C, followed by stirring at the same temperature for 3 hours. A saturated aqueous sodium hydrogencarbonate solution was added to the reaction mixture, followed by extraction with ethyl acetate. The organic layer was dried over anhydrous sodium sulfate, followed by evaporation of the solvent. The

residue was purified by preparative thin-layer chromatography (methanol : chloroform = 10:90) to obtain Compound 45 (11 mg, 37%).
$^1$H NMR ($CDCl_3$) δ (ppm): 1.41-2.08 (m, 10H), 2.72-2.81 (m, 7H), 4.69 (d, J = 5.6 Hz, 2H), 4.91 (d, J = 12.9 Hz, 2H), 5.11 (t, J = 5.9 Hz, 1H), 6.82-7.30 (m, 7H), 7.76 (s, 1H).
APCIMS m/z: $[M + H]^+$ 480

Example 46

4-(2,6-difluorobenzylamino)-5-(2-nitrophenyl)-2-(4-piperidinopiperidino)pyrimidine (Compound 46)

Compound E1 (26 mg, 0.069 mmol) obtained in Reference Example 7 was dissolved in DMF (0.14 mL), and the solution was added with 4-piperidinopyrimidine (23 mg, 0.14 mmol) and triethylamine (97 μL, 0.69 mmol), followed by stirring at 100°C for 3 hours. A saturated aqueous sodium hydrogencarbonate solution was added to the reaction mixture, followed by extraction with chloroform. The organic layer was washed with water and then dried over anhydrous sodium sulfate, followed by evaporation of the solvent. The residue was purified by preparative thin-layer chromatography (ethyl acetate : hexane : triethylamine = 25:75:10) to obtain Compound 46 (27 mg, 76%).
$^1$H NMR ($CDCl_3$) δ (ppm): 1.43-1.63 (m, 8H), 1.88-1.93 (m, 2H), 2.49-2.71 (m, 5H), 2.76-2.85 (m, 2H), 4.60-4.93 (m, 5H), 6.82-6.88 (m, 2H), 7.20-7.27 (m, 1H), 7.34 (d, J = 7.6 Hz, 1H), 7.49-7.54 (m, 1H), 7.61-7.66 (m, 2H), 7.95 (d, J = 7.9 Hz, 1H).
APCIMS m/z: $[M + H]^+$ 509

Example 47

4-(2,6-difluorobenzylamino)-5-phenyl-2-(4-piperidinopiperidino)pyrimidine (Compound 47)

Compound 47 (65 mg, 74%) was obtained using Compound E2 (62 mg) obtained in Reference Example 8 and 4-piperidinopiperidine in a similar manner to Example 46.
$^1$H-NMR ($CDCl_3$) δ (ppm): 1.38-1.73 (m, 8H), 1.81-1.95 (m, 2H), 1.40-1.68 (m, 5H), 2.80 (dt, J = 2.0, 12.0 Hz, 2H), 4.71 (d, J = 6.1 Hz, 2H), 4.85-4.97 (m, 2H), 5.34 (t, J = 6.1 Hz, 1H), 6.77-6.95 (m, 2H), 7.10-7.52 (m, 6H), 7.77 (s, 1H).
APCIMS m/z: $[M + H]^+$ 464

Example 48

4-(2,4-difluorobenzylamino)-5-(3-methyl-2-thienyl)-2-(4-piperidinopiperidino)pyrimidine (Compound 48)

Compound 48 (42 mg, 54%) was obtained using Compound E3 (57 mg) obtained in Reference Example 9 and 4-piperidinopiperidine in a similar manner to Example 46.
$^1$H-NMR ($CDCl_3$) δ (ppm): 1.42-1.62 (m ,8H), 1.86-1.91 (m, 2H), 2.04 (s, 3H), 2.47-2.56 (m, 5H), 2.80 (dt, J = 1.9, 13.0 Hz, 2H), 4.69 (d, J = 5.9 Hz, 2H), 4.88-4.93 (m, 2H), 5.10 (t, J = 5.9 Hz, 1H), 6.85 (t, J = 7.8 Hz, 2H), 6.92 (d, J = 5.1 Hz, 1H), 7.16-7.25 (m, 2H), 7.77 (s, 1H).
APCIMS m/z: $[M + H]^+$ 484

Example 49

4-(2,6-difluorobenzylamino)-2-(4-piperidinopiperidino)-5-(2-thiazoryl)pyrimidine (Compound 49)

Compound 49 (35 mg, 84%) was obtained using Compound E4 (30 mg) obtained in Reference Example 10 and 4-piperidinopiperidine in a similar manner to Example 46.
$^1$H-NMR ($CDCl_3$) δ (ppm): 1.36-1.75 (m, 8H), 1.80-1.93 (m, 2H), 2.43-2.65 (m, 5H), 2.83 (dt, J = 2.0, 12 Hz, 2H), 4.85 (d, J = 5.9 Hz, 2H), 4.83-5.03 (m, 2H), 6.78-6.93 (m, 2H), 7.04 (d, J = 3.5 Hz, 1H), 7.10-7.30 (m, 1H), 7.67 (d, J = 3.5 Hz, 1H), 8.39 (s, 1H), 9.55 (brd, 1H).
APCIMS m/z: $[M + H]^+$ 471

Example 50

4-(2,6-difluorobenzylamino)-2-(4-piperidinopiperidino)-5-(2-pyridyl)pyrimidine (Compound 50)

Compound 50 (13 mg, 100%) was obtained using Compound E5 (9.6 mg) obtained in Reference Example

11 and 4-piperidinopiperidine in a similar manner to Example 46.
$^1$H-NMR ($CDCl_3$) δ (ppm): 1.30-1.75 (m, 8H), 1.80-1.95 (m, 2H), 2.38-2.63 (m, 5H), 2.82 (dt, J = 2.0, 13 Hz, 2H), 4.86 (d, J = 5.9 Hz, 2H), 4.81-5.02 (m, 2H), 6.78-6.95 (m, 2H), 6.98-7.08 (m, 1H), 7.10-7.30 (m, 1H), 7.50-7.71 (m, 2H), 8.43 (s, 1H), 8.47 (d, J = 4.8 Hz, 1H), 10.3 (brd, 1H).
APCIMS m/z: $[M + H]^+$ 465

Example 51

4-(2,4-difluorobenzylamino)-5-(2-fluorophenyl)-2-(4-piperidinopiperidino)pyrimidine (Compound 51)

Step 1

The mixture of Compound F1a (38 mg, 0.15 mmol) obtained in Reference Example 12, 2,4-difluorobenzylamine (89 μL, 0.75 mmol) and potassium carbonate (21 mg, 0.15mmol) was stirred at 100°C for 6 hours. After the reaction mixture was cooled, water was added to the reaction mixture, followed by extraction with ethyl acetate. The organic layer was washed with 1 mol/L hydrochloric acid and saturated brine in that order and then dried over anhydrous sodium sulfate, followed by evaporation of the solvent. The residue was purified by preparative thin-layer chromatography (hexane : ethyl acetate = 7:1) to obtain 4-(2,4-difluorobenzylamino)-5-(2-fluorophenyl)-2-methylthiopyrimidine (Compound Flb) (35 mg, 65%).
$^1$H NMR ($CDCl_3$) δ (ppm): 2.51 (s, 3H), 4.69 (d, J = 5.9 Hz, 2H), 5.00-5.15 (m, 1H), 6.72-6.90 (m, 2H), 7.12-7.45 (m, 5H), 7.92 (s, 1H).
APCIMS m/z: $[M + H]^+$ 362

Step 2

Compound F1b (33 mg, 0.091 mmol) obtained in the above step was suspended in toluene (0.66 mL) and mCPBA (24 mg, 0.14 mmol) was added to the suspension, followed by stirring at room temperature for 1 hour. The reaction mixture was diluted with ethyl acetate and a saturated aqueous sodium hydrogencarbonate solution added to the reaction mixture, followed by the separation of the two liquids. The organic layer was washed with saturated brine and then dried over anhydrous sodium sulfate, followed by evaporation of the solvent. To the residue, 4-piperidinopiperidine (30 mg, 0.18 mmol) and ethanol (0.60 mL) were added. The mixture was heated to 110°C and ethanol was evaporated. The reaction mixture was heated and stirred for 2 hours and then cooled. The reaction mixture was purified by preparative thin-layer chromatography (hexane : ethyl acetate : triethylamine = 30:10:1) to obtain Compound 51 (38 mg, 86%).
$^1$H NMR ($CDCl_3$) δ (ppm): 1.35-1.78 (m, 8H), 1.80-1.92 (m, 2H), 2.39-2.63 (m, 5H), 2.79 (dt, J = 2.0, 13.0 Hz, 2H), 4.63 (d, J = 5.9 Hz, 2H), 4.73-4.95 (m, 3H), 6.70-6.89 (m, 2H), 7.08-7.41 (m, 5H), 7.80 (s, 1H).
APCIMS m/z: $[M + H]^+$ 482
Compounds 52 to 71 were obtained by reacting Compounds F1a to F3a obtained in Reference Examples 12 to 14 with corresponding amines in a similar manner to Step 1 of Reference Example 51, and treating with 3-chloroperbenzoic acid and reacting with 4-piperidinopiperidine in a similar manner to Step 2 of Reference Example 51.

Example 52

4-(2,6-dichlorobenzylamin)-5-(2-fluorophenyl)-2-(4-piperidinopiperidino)pyrimidine (Compound 52)

$^1$H-NMR ($CDCl_3$) δ (ppm): 1.35-1.70 (m, 8H), 1.78-1.95 (m, 2H), 2.48-2.68 (m, 5H), 2.84 (dt, J = 1.5, 13.0 Hz, 2H), 4.85-5.08 (m, 5H), 7.07-7.36 (m, 7H), 7.79 (s, 1H).
APCIMS m/z: $[M + H]^+$ 514

Example 53

4-(butylamino)-5-(2-fluorophenyl)-2-(4-piperidinopiperidino)pyrimidine (Compound 53)

$^1$H-NMR ($CDCl_3$) δ (ppm): 0.92 (t, J = 7.2 Hz, 3H), 1.10-1.80 (m, 12H), 1.82-1.95 (m, 2H), 2.40-2.64 (m, 5H), 2.81 (dt, J = 2.5, 13.0 Hz, 2H), 3.42 (dd, J = 13.0, 7.1 Hz, 2H), 4.50 (brd, 1H), 4.80-4.92 (m, 2H), 7.07-7.40 (m, 4H), 7.76 (s, 1H).
APCIMS m/z: $[M + H]^+$ 412

Example 54

4-(N-butyl-N-methylamino)-5-(2-fluorophenyl)-2-(4-piperidinopiperidino)pyrimidine (Compound 54)

$^1$H-NMR ($CDCl_3$) δ (ppm) : 0.84 (t, J = 7.1 Hz, 3H), 1.03-1.25 (m, 2H), 1.35-1.95 (m, 12H), 2.42-2.68 (m, 5H), 2.71 (s, 3H), 2.82 (dt, J = 1.0, 13.0 Hz, 2H), 3.29 (t, J = 7.4 Hz, 2H), 4.75-4.98 (m, 2H), 7.03-7.34 (m, 4H), 7.77 (s, 1H).
APCIMS m/z: $[M + H]^+$ 426

Example 55

5-(2-fluorophenyl)-4-(3-methylbutylamino)-2-(4-piperidinopiperidino)pyrimidine (Compound 55)

$^1$H-NMR ($CDCl_3$) δ (ppm): 0.92 (d, J = 6.6 Hz, 6H), 1.32-1.95 (m, 13H), 2.40-2.64 (m, 5H), 2.82 (dt, J = 2.1, 13.0 Hz, 2H), 3.37-3.51 (m, 2H), 4.47 (t, J = 5.1 Hz, 1H), 4.79-4.92 (m, 2H), 7.07-7.40 (m, 4H), 7.75 (s, 1H).
APCIMS m/z: $[M + H]^+$ 426

Example 56

4-butylamino-5-phenyl-2-(4-piperidinopiperidino)pyrimidine (Compound 56)

$^1$H-NMR ($CDCl_3$) δ (ppm): 0.93 (t, J = 7.2 Hz, 3H), 1.13-1.75 (m, 12H), 1.80-1.93 (m, 2H), 2.40-2.64 (m, 5H), 2.81 (dt, J = 1.0, 12.0 Hz, 2H), 3.23 (dd, J = 13.0, 7.1 Hz, 2H), 4.72-4.92 (m, 3H), 7.19-7.45 (m, 5H), 7.76 (s, 1H).
APCIMS m/z: $[M + H]^+$ 394

Example 57

4-(N-butyl-N-methylamino)-5-phenyl-2-(4-piperidinopiperidino)pyrimidine (Compound 57)

$^1$H-NMR ($CDCl_3$) δ (ppm): 0.83 (t, J = 7.1 Hz, 3H), 1.03-1.25 (m, 2H), 1.35-1.95 (m, 12H), 2.40-2.64 (m, 5H), 2.69 (s, 3H), 2.81 (dt, J = 2.3, 13.0 Hz, 2H), 3.23 (t, J = 7.6 Hz, 2H), 4.75-4.90 (m, 2H), 7.18-7.42 (m, 5H), 7.81 (s, 1H).
APCIMS m/z: $[M + H]^+$ 408

Example 58

4-(4-chloro-2-fluorobenzylamino)-5-(2,4-difluorophenyl)-2-(4-piperidinopiperidino)pyrimidine (Compound 58)

$^1$H-NMR ($CDCl_3$) δ (ppm): 1.26-1.70 (m, 8H), 1.78-1.92 (m, 2H), 2.39-2.63 (m, 5H), 2.78 (dt, J = 2.4, 13.0 Hz, 2H), 4.62 (d, J = 5.9 Hz, 2H), 4.70-4.95 (m, 3H), 6.78-7.10 (m, 4H), 7.15-7.35 (m, 2H), 7.75 (s, 1H).
APCIMS m/z: $[M + H]^+$ 516

Example 59

5-(2,4-difluorophenyl)-4-(2-nitrobenzylamino)-2-(4-piperidinopiperidino)pyrimidine (Compound 59)

$^1$H-NMR ($CDCl_3$) δ (ppm): 1.15-2.00 (m, 10H), 2.50-2.68 (m, 5H), 2.79 (dt, J = 2.0, 13 Hz, 2H), 4.65-4.82 (m, 2H), 4.88 (d, J = 6.3 Hz, 2H), 5.26 (t, J = 6.3 Hz, 1H), 6.85-7.03 (m, 2H), 7.14-7.31 (m, 1H), 7.35-7.72 (m, 3H), 7.75 (s, 1H), 8.01 (dd, J = 1.0, 7.9 Hz, 1H).
APCIMS m/z: $[M + H]^+$ 509

Example 60

5-(2,4-difluorophenyl)-4-(4-fluorobenzylamino)-2-(4-piperidinopiperidino)pyrimidine (Compound 60)

$^1$H-NMR ($CDCl_3$) δ (ppm): 1.38-1.99 (m, 10H), 2.46-2.70 (m, 5H), 2.80 (dt, J = 1.2, 13 Hz, 2H), 4.61 (d, J = 5.7 Hz, 2H), 4.71-4.90 (m, 3H), 6.85-7.10 (m, 4H), 7.16-7.40 (m, 3H), 7.77 (s, 1H).
APCIMS m/z: $[M + H]^+$ 482

Example 61

4-(2-chloro-6-fluorobenzylamino)-5-(2,4-difluorophenyl)-2-(4-piperidinopiperidino)pyrimidine (Compound 61)

APCIMS m/z: $[M + H]^+$ 516

Example 62

4-(4-chlorophenetylamino)-5-(2,4-difluorophenyl)-2-(4-piperidinopiperidino)pyrimidine (Compound 62)

$^1$H-NMR ($CDCl_3$) δ (ppm): 1.38-1.78 (m, 8H), 1.85-1.99 (m, 2H), 2.43-2.65 (m, 5H), 2.75-2.93 (m, 4H), 3.62 (q, J = 5.9 Hz, 2H), 4.43 (d, J = 5.9 Hz, 1H), 4.79-4.92 (m, 2H), 6.82-6.93 (m, 2H), 7.02-7.32 (m, 5H), 7.73 (s, 1H).
APCIMS m/z: $[M + H]^+$ 512

Example 63

4-(2-chlorophenetylamino)-5-(2,4-difluorophenyl)-2-(4-piperidinopiperidino)pyrimidine (Compound 63)

$^1$H-NMR ($CDCl_3$) δ (ppm): 1.35-1.73 (m, 8H), 1.85-1.99 (m, 2H), 2.44-2.66 (m, 5H), 2.75-2.93 (m, 2H), 3.02 (t, J = 6.0 Hz, 2H), 3.68 (q, J = 6.0 Hz, 2H), 4.50 (d, J = 6.0 Hz, 1H), 4.82-4.93 (m, 2H), 6.82-6.93 (m, 2H), 7.10-7.37 (m, 5H), 7.72 (s, 1H).
APCIMS m/z: $[M + H]^+$ 512

Example 64

4-(2,4-dichlorophenetylamino)-5-(2,4-difluorophenyl)-2-(4-piperidinopiperidino)pyrimidine (Compound 64)

$^1$H-NMR ($CDCl_3$) δ (ppm): 1.34-1.75 (m, 8H), 1.85-2.00 (m, 2H), 2.43-2.67 (m, 5H), 2.84 (dt, J = 1.2, 12.0 Hz, 2H), 3.00 (t, J = 6.1 Hz, 2H), 3.66 (q, J = 6.1 Hz, 2H), 4.46 (d, J = 6.1 Hz, 1H), 4.80-4.98 (m, 2H), 6.81-6.99 (m, 2H), 7.02-7.22 (m, 3H), 7.36 (d, J = 2.0 Hz, 1H), 7.75 (s, 1H).
APCIMS m/z: $[M + H]^+$ 546

Example 65

5-(2,4-difluorophenyl)-4-(4-fluorophenetylamino)-2-(4-piperidinopiperidino)pyrimidine (Compound 65)

$^1$H-NMR ($CDCl_3$) δ (ppm): 1.35-1.77 (m, 8H), 1.84-2.00 (m, 2H), 2.40-2.70 (m, 5H), 2.73-2.98 (m, 4H), 3.62 (q, J = 5.9 Hz, 2H), 4.42 (d, J = 5.9 Hz, 1H), 4.79-4.96 (m, 2H), 6.80-7.25 (m, 7H), 7.75 (s, 1H).
APCIMS m/z: $[M + H]^+$ 596

Example 66

5-(2,4-difluorophenyl)-4-(2,2-diphenylethylamino)-2-(4-piperidinopiperidino)pyrimidine (Compound 66)

APCIMS m/z: $[M + H]^+$ 554

Example 67

5-(2,4-difluorophenyl)-4-(3,3-dimethylbutylamino)-2-(4-piperidinopiperidino)pyrimidine (Compound 67)

APCIMS m/z: $[M + H]^+$ 458

Example 68

4-(4-tert-butylcyclohexylamino)-5-(2,4-difluorophenyl)-2-(4-piperidinopiperidino)pyrimidine (Compound 68)

APCIMS m/z: $[M + H]^+$ 512

Example 69

4-(N-butyl-N-methylamino)-5-(2,4-difluorophenyl)-2-(4-piperidinopiperidino)pyrimidine (Compound 69)

APCIMS m/z: $[M + H]^+$ 444

Example 70

5-(2,4-difluorophenyl)-4-(4-methanesulfonylbenzylamino)-2-(4-piperidinopiperidino)pyrimidine (Compound 70)

APCIMS m/z: $[M + H]^+$ 542

Example 71

4-(2,6-dichlorophenetylamino)-5-(2,4-difluorophenyl)-2-(4-piperidinopiperidino)pyrimidine (Compound 71)

APCIMS m/z: $[M + H]^+$ 546

Example 72

4-(2,4-difluorobenzylamino)-5-(2,4-difluorophenyl)-2-[4-(1-methylpiperidinio)piperidino]pyrimidine iodide (Compound 72)

Compound 2 (1.43 g) obtained in Example 2 was dissolved in dichloromethane (11 mL), and the solution was added with methyl iodide (0.712 mL, 11.4 mmol), followed by stirring at room temperature for 24 hours. The reaction mixture was concentrated and the residue was reslurried with ethanol for 2 hours, whereby crystals were precipitated. The crystals were collected by filtration and then dried to obtain Compound 72 (1.26 g, 69%).
$^1$H NMR ($CDCl_3$) $\delta$ (ppm): 1.62-2.02 (m, 8H), 2.26 (brd, J = 11.5 Hz, 2H), 3.08 (s, 3H), 3.12 (brt, J = 12.5 Hz, 2H), 3.73-3.77 (m, 4H), 4.48-4.57 (m, 1H), 4.60 (d, J = 5.9 Hz, 2H), 4.91-4.99 (m, 3H), 6.75-7.00 (m, 4H), 7.20-7.34 (m, 2H), 7.72 (s, 1H).
APCIMS m/z: $[M + H]^+$ 642

Example 73

4-(2,4-difluorobenzylamino)-5-(2,4-difluorophenyl)-2-[4-(1-ethylpiperidinio)piperidino]pyrimidine iodide (Compound 73)

Compound 73 (81.3 mg, 31%) was obtained using Compound 2 (0.198 g) obtained in Example 2 and ethyl iodide (1.59 mL, 19.9 mmol) in a similar manner to Example 72.
$^1$H-NMR ($CDCl_3$) $\delta$ (ppm): 1.44 (t, J = 6.3 Hz, 3H), 1.63-1.93 (m, 4H), 1.93-2.13 (m, 4H), 2.17-2.36 (m, 2H), 3.14 (brt, J = 12.5 Hz, 2H), 3.48-3.66 (m, 6H), 4.16-4.24 (m, 1H), 4.61 (brd, J = 5.9 Hz, 2H), 4.95 (brd, J = 12.5 Hz, 2H), 5.03 (t, J = 6.0 Hz, 1H), 6.76-7.00 (m, 4H), 7.22-7.37 (m, 2H), 7.70 (s, 1H).
APCIMS m/z: $[M + H]^+$ 656

Example 74

4-(2,4-difluorobenzylamino)-5-(2,4-difluorophenyl)-2-[4-(1-propylpiperidinio)piperidino]pyrimidine iodide (Compound 74)

Compound 74 (43.2 mg, 17%) was obtained using Compound 2 (0.188 g) obtained in Example 2 and propyl iodide (0.81 mL, 8.31 mmol) in a similar manner to Example 72.
$^1$H-NMR ($CDCl_3$) $\delta$ (ppm): 1.06 (t, J = 7.2 Hz, 3H), 1.70-2.12 (m, 10H), 2.26 (brd, J = 11.0 Hz, 2H), 3.17 (brt, J = 12.3Hz, 2H), 3.32-3.38 (m, 2H), 3.59-3.63 (m, 4H), 4.25-4.34 (m, 1H), 4.61 (d, J = 5.8 Hz, 2H), 4.96 (brd, J = 13.5 Hz, 2H), 5.06 (t, J = 5.9 Hz, 1H), 6.75-7.01 (m, 4H), 7.21-7.37 (m, 2H), 7.71 (s, 1H).
APCIMS m/z: $[M + H]^+$ 670

Example 75

2-[4-(1-allylpiperidinio)piperidino]-4-(2,4-difluorobenzylamino)-5-(2,4-difluorophenyl)pyrimidine bromide (Compound 75)

Compound 75 (0.198 g, 74%) was obtained using Compound 2 (0.214 g) obtained in Example 2 and allyl bromide (0.816 mL, 9.44 mmol) in a similar manner to Example 72.
$^1$H-NMR ($CD_3OD$) δ (ppm): 1.70-1.90 (m, 4H), 1.91-2.18 (m, 4H), 2.28 (brd, J = 10.6 Hz, 2H), 3.06 (brt, J = 12.2 Hz, 2H), 3.50-3.67 (m, 4H), 4.07-4.23 (m, 3H), 4.61 (brd, J = 5.9 Hz, 2H), 4.93 (brd, J = 13.2 Hz, 2H), 5.16 (t, J = 5.8 Hz, 1H), 5.67 (d, J = 10.2 Hz, 1H), 5.78 (d, J = 16.8 Hz, 1H), 5.98-6.13 (m, 1H), 6.75-7.00 (m, 4H), 7.22-7.40 (m, 2H), 7.69 (s, 1H).
FABMS m/z: $[M - Br]^+$ 540

Example 76

4-(2,4-difluorobenzylamino)-5-(2,4-difluorophenyl)-2-[4-(1,2-dimethylpiperidinio)piperidino]pyrimidine iodide (Compound 76)

Compound 76 (0.596 g, 58%) was obtained using Compound 3 (0.813 g) obtained in Example 3 and methyl iodide (1.97 mL, 31.6 mmol) in a similar manner to Example 72.
$^1$H-NMR (DMSO-$d_6$) δ (ppm): 1.25 (d, J = 6.4 Hz, 3H), 1.42-1.89 (m, 2H), 2.05-2.09 (m, 2H), 2.81-2.94 (m, 2H), 2.95 (s, 3H), 3.03-3.11 (m, 1H), 3.22 (brd, J = 12.8 Hz, 1H), 3.66-3.73 (m, 1H), 3.88 (brd, J = 11.3 Hz, 1H), 4.48 (brd, J = 11.3 Hz, 2H), 4.73 (brd, J = 5.7 Hz, 2H), 7.03-7.43 (m, 7H), 7.69 (s, 1H).
APCIMS m/z: $[M + H]^+$ 656

Example 77

4-(2,4-difluorobenzylamino)-5-(2,4-difluorophenyl)-2-[4-(1,3-dimethylpiperidinio)piperidino]pyrimidine iodide (Compound 77)

Compound 77 (0.659 g, 58%) was obtained using Compound 1 (0.881 g) obtained in Example 1 and methyl iodide (2.13 mL, 34.2 mmol) in a similar manner to Example 72.
$^1$H-NMR (DMSO-$d_6$) δ (ppm): 0.88-0.91 (m, 3H), 1.04-1.13 (m, 1H), 1.49-2.23 (m, 9H), 2.72-2.90 (m, 6H), 3.10 (brt, J = 11.5 Hz, 1H), 3.37-3.46 (m, 2H), 3.56-3.71 (m, 1H), 4.47 (brd, J = 5.5 Hz, 2H), 4.77 (brd, J = 12.5 Hz, 2H), 7.02-7.25 (m, 4H), 7.31-7.42 (m, 3H), 7.68 (s, 1H).
APCIMS m/z: $[M - I]^+$ 528

Formulation Example 1 (Tablet)

Tablets having the following compositions are prepared according to a conventional method.

| Formulation | Compound 3 | 20 mg |
|---|---|---|
| | Lactose<br>Potato starch<br>Hydroxypropyl cellulose<br>Magnesium stearate | 143.4 mg<br>30 mg<br>6 mg<br>0.6 mg |
| | | 200 mg |

Formulation Example 2 (Injection)

An injection having the following composition is prepared according to a conventional method.

| Formulation | Compound 40 | 2 mg |
|---|---|---|
| | Purified soybean oil<br>Purified egg yolk lecithin | 200 mg<br>24 mg |

(continued)

| | | |
|---|---|---|
| Formulation | Compound 40 | 2 mg |
| | Glycerin for injection<br>Distilled water for injection | 50 mg<br>1.72 mL |
| | | 2.00 mL |

Industrial Applicability

The present invention provides a pyrimidine derivative, or quaternary ammonium salts thereof, or pharmaceutically acceptable salts thereof which have anti-inflammatory activities (for example, cellular infiltration inhibitory activities, etc), modulating activities on TARC and/or MDC functions (for example, inhibitory activities against binding of TARC and/or MDC to T cells, etc) and the like, and are useful for treating and/or preventing diseases which is related to T cells such as allergic diseases, autoimmune diseases, transplant rejection, etc. as well as preventing cancer metastasis. Examples of the diseases which is related to T-cells include asthma, allergic rhinitis, chronic rhinitis, pollinosis, conjunctivitis, urticaria, psoriasis, atopic dermatitis, cutaneous pruritus, cutaneous candidiasis, oral candidiasis, articular rheumatism, various collagen diseases, systemic lupus erythematodes, Sjögren syndrome, cellular rejection after organ transplantation, cancer, leukemia including adult T cell leukemia (ATL), eosinophilic pneumonia, eosinophilic sinusitis, rhinitis with eosinophilia, interstitial cystitis, endometriosis, Churg-Strauss syndrome, septicemia, pain, neuralgia, insulin-dependent diabetes mellitus (IDDM), mycosis fungoides, contact dermatitis, cutaneous T cell lymphoma and the like.

## Claims

**1.** A pyrimidine derivative represented by Formula (I)

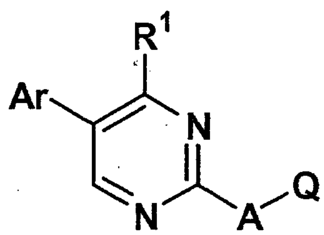

(I)

[wherein Ar represents substituted or unsubstituted aryl or a substituted or unsubstituted heteroaromatic group, $R^1$ represents $-NR^2R^3$ (wherein $R^2$ represents a hydrogen atom or substituted or unsubstituted lower alkyl, and $R^3$ represents substituted or unsubstituted lower alkyl, substituted or unsubstituted aralkyl or substituted or unsubstituted heteroaromatic alkyl) or Formula (II)

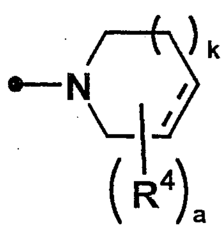

(II)

(wherein --- represents a single or double bond, k represents an integer of 0 to 4, a represents a number ranging from 0 to a substitutable number, $R^4$ represents substituted or unsubstituted lower alkyl, and each $R^4$ may be the same or different when a is 2 or more),
A represents a single bond, Formula (III)

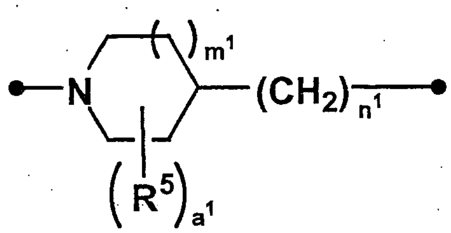

(III)

(wherein $m^1$ represents an integer of 0 to 2, $n^1$ represents an integer of 0 to 4, $a^1$ represents a number ranging from 0 to a substitutable number, $R^5$ represents substituted or unsubstituted lower alkyl, substituted or unsubstituted aralkyl, a substituted or unsubstituted heteroalicyclic group, or substituted or unsubstituted heteroaromatic alkyl, and each $R^5$ may be the same or different when $a^1$ is 2 or more) or Formula (IV)

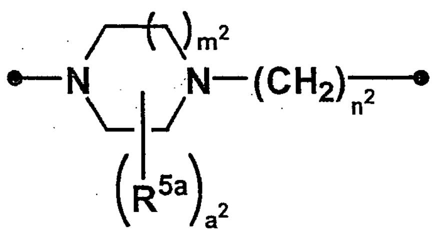

(IV)

(wherein $a^2$ represents a number ranging from 0 to a substitutable number, and $m^2$, $n^2$ and $R^{5a}$ have the same meanings as $m^1$, $n^1$, and $R^5$ defined above, respectively), and

(a) when A represents Formula (III) or (IV),
Q represents $-NR^6R^7$ (wherein $R^6$ and $R^7$ may be the same or different and each represents a hydrogen atom, substituted or unsubstituted lower alkyl, substituted or unsubstituted aralkyl, a substituted or unsubstituted heteroalicyclic group, substituted or unsubstituted heteroalicyclic alkyl or substituted or unsubstituted heteroaromatic alkyl) or a substituted or unsubstituted heteroalicyclic group,
(b) when A represents a single bond,
Q represents substituted or unsubstituted piperidin-4-ylamino, substituted or unsubstituted perhydroazepin-4-ylamino or $-N(R^8)-(CH_2)_p-NR^9R^{10}$ (wherein p represents 1, 2 or 4, $R^8$ represents a hydrogen atom or substituted or unsubstituted lower alkyl, and $R^9$ and $R^{10}$ may be the same or different and each represents a hydrogen atom, substituted or unsubstituted lower alkyl, substituted or unsubstituted aralkyl, a substituted or unsubstituted heteroalicyclic group, substituted or unsubstituted heteroalicyclic alkyl or substituted or unsubstituted heteroaromatic alkyl, or $R^9$ and
$R^{10}$ are combined together with the adjacent nitrogen atom thereto to form a substituted or unsubstituted heteroalicyclic group], or a quaternary ammonium salt thereof, or a pharmaceutically acceptable salt thereof.

**2.** The pyrimidine derivative, or the quaternary ammonium salt thereof, or the pharmaceutically acceptable salt thereof according to Claim 1, wherein $R^1$ is $-NR^2R^{3A}$ (wherein $R^2$ has the same meaning as defined above, and $R^{3A}$ represents substituted or unsubstituted aralkyl or substituted or unsubstituted heteroaromatic alkyl).

**2.** The pyrimidine derivative, or the quaternary ammonium salt thereof, or the pharmaceutically acceptable salt thereof according to Claim 2, wherein $R^{3A}$ is substituted aralkyl.

**4.** The pyrimidine derivative, or the quaternary ammonium salt thereof, or the pharmaceutically acceptable salt thereof according to any of Claims 1 to 3, wherein A represents Formula (III)

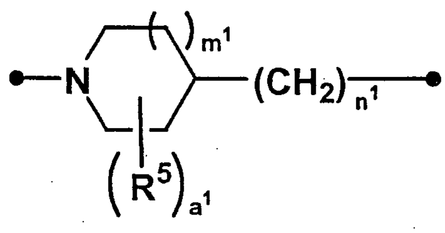

(III)

(wherein $m^1$, $n^1$, $a^1$ and $R^5$ have the same meanings as defined above, respectively) or Formula (IV)

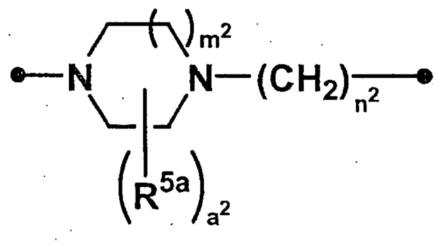

(IV)

(wherein $m^2$, $n^2$, $a^2$ and $R^{5a}$ have the same meanings as defined above, respectively).

**5.** The pyrimidine derivative, or the quaternary ammonium salt thereof, or the pharmaceutically acceptable salt thereof according to Claim 4, wherein $m^1$ is 1 or 2 and $m^2$ is 1.

**6.** The pyrimidine derivative, or the quaternary ammonium salt thereof, or the pharmaceutically acceptable salt thereof according to any of Claims 1 to 3, wherein A is Formula (IIIA)

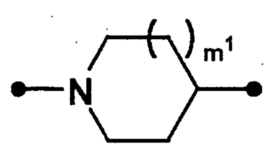

(IIIA)

(wherein $m^1$ has the same meaning as defined above).

**7.** The pyrimidine derivative, or the quaternary ammonium salt thereof, or the pharmaceutically acceptable salt thereof according to Claim 6, wherein $m^1$ is 1.

**8.** The pyrimidine derivative, or the quaternary ammonium salt thereof, or the pharmaceutically acceptable salt thereof according to any of Claims 1 to 7, wherein Q is $-NR^6R^7$ (wherein $R^6$ and $R^7$ have the same meanings as defined above, respectively), substituted or unsubstituted 1,2,5,6-tetrahydropyridin-1-yl, substituted or unsubstituted 1,2,3,4-tetrahydroisoquinolin-2-yl or Formula (V)

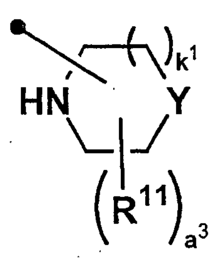

(V)

{wherein $k^1$ represents an integer of 1 to 4, $a^3$ represents a number ranging 0 to a substitutable number, Y represents a single bond, -S-, -O-, -C(=O)- or -N($R^{12}$)- (wherein $R^{12}$ represents a hydrogen atom or substituted or unsubstituted alkanoyl), $R^{11}$ represents a hydrogen atom, substituted or unsubstituted lower alkyl, substituted or unsubstituted lower alkenyl, substituted or unsubstituted lower alkynyl, substituted or unsubstituted mono(lower alkyl)amino, substituted or unsubstituted di(lower alkyl)amino, substituted or unsubstituted lower alkoxy, hydroxy, substituted or unsubstituted lower alkanoyl, substituted or unsubstituted aryl, substituted or unsubstituted aralkyl, substituted or unsubstituted aryloxy, substituted or unsubstituted aralkyloxy, substituted or unsubstituted aroyl, substituted or unsubstituted lower alkoxycarbonyl, substituted or unsubstituted mono(lower alkyl)aminocarbonyl, substituted or unsubstituted di(lower alkyl)aminocarbonyl, cyano, carboxy, carbamoyl, amino, nitro, substituted or unsubstituted lower alkylsulfinyl, substituted or unsubstituted lower alkylsulfonyl, mercapto or lower alkylthio, and when $a^3$ is 2 or more, each $R^{11}$ may be the same or different.

**9.** The pyrimidine derivative, or the quaternary ammonium salt thereof, or the pharmaceutically acceptable salt thereof according to any of Claims 1 to 7, wherein Q is substituted or unsubstituted piperidino.

**10.** The pyrimidine derivative, or the quaternary ammonium salt thereof, or the pharmaceutically acceptable salt thereof according to any of Claims 1 to 3, wherein A is a single bond.

**11.** The pyrimidine derivative, or the quaternary ammonium salt thereof, or the pharmaceutically acceptable salt thereof according to any of Claims 1 to 10, wherein Ar is substituted or unsubstituted aryl.

**12.** The pyrimidine derivative, or the quaternary ammonium salt thereof, or the pharmaceutically acceptable salt thereof according to any of Claims 1 to 10, wherein Ar is substituted or unsubstituted phenyl, substituted or unsubstituted naphthyl, substituted or unsubstituted biphenyl, substituted or unsubstituted thienyl, substituted or unsubstituted thiazolyl, substituted or unsubstituted benzofuranyl, substituted or unsubstituted furyl or substituted or unsubstituted pyridyl.

**13.** The pyrimidine derivative, or the quaternary ammonium salt thereof, or the pharmaceutically acceptable salt thereof according to any of Claims 1 to 12, wherein $R^2$ is a hydrogen atom.

**14.** The pyrimidine derivative, or the quaternary ammonium salt thereof, or the pharmaceutically acceptable salt thereof according to any of Claims 1 to 13, wherein the quaternary ammonium salt is formed by addition of Z-El (wherein Z represents substituted or unsubstituted lower alkyl, substituted or unsubstituted lower alkenyl or substituted or unsubstituted lower aralkyl and E1 represents a leaving group) to a nitrogen atom in Q.

**15.** The quaternary ammonium salt of the pyrimidine derivative or the pharmaceutically acceptable salt thereof according to Claim 14.

**16.** A pharmaceutical composition which comprises, as an active ingredient, the pyrimidine derivative, or the quaternary ammonium salt thereof, or the pharmaceutically acceptable salt thereof according to any of Claims 1 to 14.

**17.** An anti-inflammatory agent which comprises, as an active ingredient, the pyrimidine derivative, or the quaternary ammonium salt thereof, or the pharmaceutically acceptable salt thereof according to any of Claims 1 to 14.

**18.** A therapeutic and/or preventive agent for a disease which is relared to thymus and activation-regulated chemokine (TARC) and/or macrophage-derived chemokine (MDC) which comprises, as an active ingredient, the pyrimidine derivative, or the quaternary ammonium salt thereof, or the pharmaceutically acceptable salt thereof according to any of Claims 1 to 14.

**19.** A pharmaceutical composition which comprises, as an active ingredient, the quaternary ammonium salt of the pyrimidine derivative or the pharmaceutically acceptable salt thereof according to Claim 15.

**20.** An anti-inflammatory agent which comprises, as an active ingredient, the quaternary,ammonium salt of the pyrimidine derivative or the pharmaceutically acceptable salt thereof according to Claim 15.

**21.** A therapeutic and/or preventive agent for a disease which is relared to thymus and activation-regulated chemokine (TARC) and/or macrophage-derived chemokine (MDC) which comprises, as an active ingredient, the quaternary ammonium salt of the pyrimidine derivative or the pharmaceutically acceptable salt thereof according to Claim 15.

**22.** Use of the pyrimidine derivative, or the quaternary ammonium salt thereof, or the pharmaceutically acceptable salt thereof according to any of Claims 1 to 14, for the manufacture of an anti-inflammatory agent.

**23.** Use of the pyrimidine derivative, or the quaternary ammonium salt thereof, or the pharmaceutically acceptable salt thereof according to any of Claims 1 to 14, for the manufacture of a therapeutic and/or preventive agent for a disease which is relared to thymus and activation-regulated chemokine (TARC) and/or macrophage-derived chemokine (MDC).

**24.** Use of the quaternary ammonium salt of the pyrimidine derivative or the pharmaceutically acceptable salt thereof according to Claim 15, for the manufacture of an anti-inflammatory agent.

**25.** Use of the quaternary ammonium salt of the pyrimidine derivative or the pharmaceutically acceptable salt thereof according to Claim 15, for the manufacture of a therapeutic and/or preventive agent for a disease which is relared to thymus and activation-regulated chemokine (TARC) and/or macrophage-derived chemokine (MDC).

**26.** A method for treating and/or preventing inflammation, which comprises administering an effective amount of the pyrimidine derivative, or the quaternary ammonium salt thereof, or the pharmaceutically acceptable salt thereof according to any of Claims 1 to 14.

**27.** A method for treating and/or preventing a disease which is relared to thymus and activation-regulated chemokine (TARC) and/or macrophage-derived chemokine (MDC), which comprises administering an effective amount of the pyrimidine derivative, or the quaternary ammonium salt thereof, or the pharmaceutically acceptable salt thereof according to any of Claims 1 to 14.

**28.** A method for treating and/or preventing inflammation, which comprises administering an effective amount of the quaternary ammonium salt of the pyrimidine derivative or the pharmaceutically acceptable salt thereof according to Claim 15.

**29.** A method for treating and/or preventing a disease which is relared to thymus and activation-regulated chemokine (TARC) and/or macrophage-derived chemokine (MDC), which comprises administering an effective amount of the quaternary ammonium salt of the pyrimidine derivative or the pharmaceutically acceptable salt thereof according to Claim 15.

**INTERNATIONAL SEARCH REPORT**

International application No.
PCT/JP2004/001962

A. CLASSIFICATION OF SUBJECT MATTER
Int.Cl[7] C07D239/42, 401/04, 401/14, 405/14, 409/14, 417/14, A61K31/506, A61P29/00, 37/06, 37/08, 43/00

According to International Patent Classification (IPC) or to both national classification and IPC

B. FIELDS SEARCHED

Minimum documentation searched (classification system followed by classification symbols)
Int.Cl[7] C07D239/42, 401/04, 401/14, 405/14, 409/14, 417/14, A61K31/506

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 99/65897 A1 (CHIRON CORP.), 23 December, 1999 (23.12.99), Claims & EP 1087963 A1 | 1-3,10-13,16 |
| A | WO 02/83693 A1 (ASTRAZENECA AB.), 24 October, 2002 (24.10.02), Full text (Family: none) | 1-25 |
| A | WO 98/06398 A1 (SMITHKLINE BEECHAM CORP.), 19 February, 1998 (19.02.98), Full text & EP 939634 A1 & JP 12-516620 A | 1-25 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 April, 2004 (13.04.04) | 27 April, 2004 (27.04.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/001962

**Box No. II Observations where certain claims were found unsearchable (Continuation of item 2 of first sheet)**

This international search report has not been established in respect of certain claims under Article 17(2)(a) for the following reasons:

1. [X] Claims Nos.: 26 to 29
   because they relate to subject matter not required to be searched by this Authority, namely:
   It pertains to methods for treatment of the human body by therapy.

2. [ ] Claims Nos.:
   because they relate to parts of the international application that do not comply with the prescribed requirements to such an extent that no meaningful international search can be carried out, specifically:

3. [ ] Claims Nos.:
   because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)